# EUROPEAN PATENT SPECIFICATION

(11) **EP 1 910 378 B1**
(45) Date of publication and mention of the grant of the patent: **20.06.2012**
(21) Application number: 06761122.8
(22) Date of filing: 17.07.2006
(51) Int. Cl.: C07D 495/04, A61K 31/4709, A61K 31/4365, A61P 31/12, C07D 215/22, C07D 401/12, C07D 413/04

(54) **HEPATITIS C INHIBITOR PEPTIDE ANALOGS**
PEPTIDANALOGE ALS HEPATITIS C-HEMMER
ANALOGUES DE PEPTIDES INHIBITEURS DE L'HEPATITE C

(30) Priority: 20.07.2005 US 700950 P
(43) Date of publication of application: 16.04.2008
(73) Proprietor: Boehringer Ingelheim International GmbH, 55216 Ingelheim am Rhein (DE)
(72) Inventor: BAILEY, Murray, D., Laval, Québec H7S 2G5 (CA); FORGIONE, Pasquale, Laval, Québec H7S 2G5 (CA); LLINAS-BRUNET, Montse, Laval, Québec H7S 2G5 (CA); POUPART, Marc-André, Laval, Québec H7S 2G5 (CA)
(74) Representative: Hammann, Heinz
(86) International application number: PCT/CA2006/001160
(87) International publication number: WO 2007/009227

(56) References cited:
- EP-B- 1 505 945
- WO-A-03/099274
- WO-A-2006/000085
- WO-A1-03/099274
- WO-A1-2006/000085
- WO-A2-02/060926
- CA-A1- 2 474 156
- US-A- 4 883 914
- US-A1- 2004 077 551
- US-A1- 2004 077 551

## Description

### FIELD OF THE INVENTION

The present invention relates to compounds, compositions and methods for the treatment of hepatitis C virus (HCV) infection. In particular, the present invention provides novel peptide analogs, pharmaceutical compositions containing such analogs and methods for using these analogs in the treatment of HCV infection.

### BACKGROUND OF THE INVENTION

Hepatitis C virus (HCV) is the major etiological agent of post-transfusion and community-acquired non-A non-B hepatitis worldwide. It is estimated that over 200 million people worldwide are infected by the virus. A high percentage of carriers become chronically infected and many progress to chronic liver disease, so-called chronic hepatitis C. This group is in turn at high risk for serious liver disease such as liver cirrhosis, hepatocellular carcinoma and terminal liver disease leading to death.

The mechanism by which HCV establishes viral persistence and causes a high rate of chronic liver disease has not been thoroughly elucidated. It is not known how HCV interacts with and evades the host immune system. In addition, the roles of cellular and humoral immune responses in protection against HCV infection and disease have yet to be established. Immunoglobulins have been reported for prophylaxis of transfusion-associated viral hepatitis; however, the Center for Disease Control does not presently recommend immunoglobulin treatment for this purpose. The lack of an effective protective immune response is hampering the development of a vaccine or adequate post-exposure prophylaxis measures, so in the near-term, hopes are firmly pinned on antiviral interventions.

Various clinical studies have been conducted with the goal of identifying pharmaceutical agents capable of effectively treating HCV infection in patients afflicted with chronic hepatitis C. These studies have involved the use of interferon-alpha, alone and in combination with other antiviral agents. Such studies have shown that a substantial number of the participants do not respond to these therapies, and of those that do respond favorably, a large proportion were found to relapse after termination of treatment.

Interferon in combination with ribavirin has been approved for the treatment of patients with chronic hepatitis C. However, side effects caused by IFN (such as retinopathy, thyroiditis, acute pancreatitis, depression) are not alleviated with this combination therapy. Pegylated forms of interferons such as PEG-Intron® and Pegasys® can apparently partially address these deleterious side effects but antiviral drugs still remain the avenue of choice for oral treatment of HCV.

Therefore, a need exists for the development of effective antiviral agents for treatment of HCV infection that overcome the limitations of existing pharmaceutical therapies.

HCV is an enveloped positive strand RNA virus in the Flaviviridae family. The single strand HCV RNA genome is approximately 9500 nucleotides in length and has a single open reading frame (ORF) encoding a single large polyprotein of about 3000 amino acids. In infected cells, this polyprotein is cleaved at multiple sites by cellular and viral proteases to produce the structural and non-structural (NS) proteins. In the case of HCV, the generation of mature nonstructural proteins (NS2, NS3, NS4A, NS4B, NS5A, and NS5B) is effected by two viral proteases. The first (generally referred to as the NS2/3 protease) cleaves at the NS2-NS3 junction; the second (the NS3 protease) is a serine protease contained within the N-terminal region of NS3 and mediates all the subsequent cleavages downstream of NS3, both in *cis,* at the NS3-NS4A cleavage site, and in *trans,* for the remaining NS4A-NS4B, NS4B-NS5A, NS5A-NS5B sites. The NS4A protein appears to serve multiple functions, acting as a cofactor for the NS3 protease and possibly assisting in the membrane localization of NS3 and other viral replicase components. The complex formation of the NS3 protease with NS4A seems necessary to the processing events, enhancing the proteolytic efficiency at all of the sites. The NS3 protein also exhibits nucleoside triphosphatase and RNA helicase activities. NS5B is a RNA-dependent RNA polymerase that is involved in the replication of HCV.

A general strategy for the development of antiviral agents is to inactivate virally encoded enzymes that are essential for the replication of the virus. In a two day clinical trial, it has been shown that the HCV NS3 protease inhibitor BILN 2061 is effective in rapidly reducing viral loads in patients infected with the hepatitis C virus (Gastroenterology (2004) 127(5): 1347-1355), thus providing proof of principle of the clinical antiviral activity of HCV NS3 protease inhibitors.

The NS3 protease has been found to potentially have an additional impact by blocking the IFN-mediated cellular antiviral activity in the infected cell (Foy et al., Science, 17 April.2003). This lends credence to a hypothesis that the NS3/NS4A protease may represent a dual therapeutic target, the inhibition of which may both block viral replication and restore interferon response of HCV infected cells.

Inhibitors of the HCV NS3 protease have been described in WO 00/09543 (Boehringer Ingelheim), WO 03/064456 (Boehringer Ingelheim), WO 03/064416 (Boehringer Ingelheim), WO 2004/101602 (Boehringer Ingelheim), WO 2004/101605 (Boehringer Ingelheim), WO 2004/103996 (Boehringer Ingelheim), WO 02/060926 (Bristol-Myers Squibb), WO 03/099316 (Bristol-Myers Squibb), WO 03/099274 (Bristol-Myers Squibb), WO 2004/032827 (Bristol-Myers Squibb), WO 2004/043339 (Bristol-Myers Squibb), WO 2005/051410 (Bristol-Myers Squibb) and WO 2005/054430 (Bristol-Myers Squibb).

Peptide analog hepatitis C inhibitors have been described in WO 03/099274. However, the compounds provided by this invention exhibit unexpected advantages. In general they show one or more of the following advantages:
lower IC₅₀ values in a NS3-NS4A protease enzymatic assay; and/or
lower EC₅₀ values in a cell based HCV RNA replication assay.

### SUMMARY OF THE INVENTION

Included in the scope of the invention are compounds of formula (I): 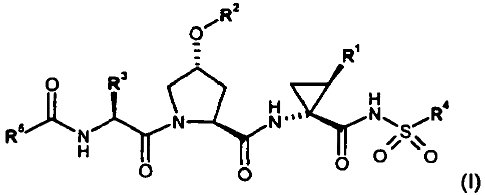 wherein
- **R¹**: is selected from (C₂₋₄)alkenyl and (C₂₋₄)alkyl;
- **R²**: is a group of formula: 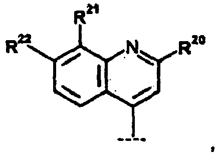 wherein
**R²⁰** is selected from -O-(C₁₋₆₁)alkyl and -S-(C₁₋₆)alkyk;
**R²¹** is selected from H, (C₁₋₈)alkyl, halogen, -O-(C₁₋₆)alkyl and -S-(C₁₋₆)alkyl; and
**R²²** is H or -O-(C₁₋₄)alkyl;
- **R³**: is (C₁₋₆)alkyl;
- **R⁴**: is (C₃₋₈)cycloalkyl, optionally substituted with (C₁₋₆)alkyl; and
- **R⁵**: is **R⁵⁰**-O- or **R⁶⁰**-NH-; wherein **R⁶⁰** is (C₁₋₆)alkyl or (C₃₋₇)cycloalkyl; wherein the (C₃₋₇)cycloalkyl is optionally substituted with (C₁₋₆)alkyl; or a salt thereof.

An advantage of one aspect of the present invention resides in the fact that compounds according to this invention specifically inhibit the NS3 protease and do not show significant inhibitory activity against other serine proteases such as human leukocyte elastase (HLE) or cysteine proteases such as human liver cathepsin B (Cat B).

Another aspect of this invention provides a compound of formula (I) ora pharmaceutically acceptable salt thereof, as a medicament

Still another aspect of this-invention provides a pharmaceutical composition comprising a therapeutically effective amount of a compound of formula (I) or a pharmaceutically acceptable salt thereof; and one or more pharmaceutically acceptable carriers.

According to an embodiment of this aspect, the pharmaceutical composition according to this invention additionally comprises at least one other antiviral agent

An additional aspect of this invention refers to an article of manufacture comprising a composition effective to treat a hepatitis C viral infection; and packaging material comprising a label which indicates that the composition can be used to treat infection by the hepatitis C virus; wherein the composition comprises a compound of formula (I) according to this invention or a pharmaceutically acceptable salt thereof.

### DETAILED DESCRIPTION OF PREFERRED EMBODIMENTS

### Definitions

As used herein, the following definitions apply unless otherwise noted:

With reference to the instances where (*R*) or (*S*) is used to designate the absolute configuration of a substituent or asymmetric center of a compound of formula I, the designation is done in the context of the whole compound and not in the context of the substituent or asymmetric center alone.

The designations "P3, P2, P1 and P1' " as used herein refer to the position of the amino acid residues starting from the N-terminus of the peptide analogs and extending towards and beyond the cleavage site, i.e. the bond in a substrate of the protease enzyme which is normally cleaved by the catalytic action of the protease enzyme. Thus, P3 refers to position 3 from the C-terminal side of the cleavage site. P2: position 2 from the C-terminal side of the cleavage site, etc.. The bond between the P1 and P1' residues corresponds to the cleavage site. Thus, the P1' position corresponds to the first position on the N-terminal side of the cleavage site (see Berger A. & Schechter I., Transactions of the Royal Society London series B257, 249-264 (1970)). In the context of the compounds of formula (I) herein described, these positions are as designated in the following formula:

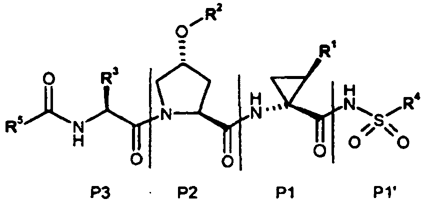

The term "(C₁₋ₙ)alkyl" as used herein, wherein n is an integer, either alone or in combination with another substituent, means acyclic, straight or branched chain alkyl substituents containing from 1 to n carbon atoms. "(C₁₋₆)alkyl" includes, but is not limited to, methyl, ethyl, n-propyl, n-butyl, 1-methylethyl (*iso*-propyl), 1-methylpropyl, 2-methylpropyl, 1,1-dimethylethyl (*tert*-butyl), pentyl and hexyl. The abbreviation Me denotes a methyl group; Et denotes an ethyl group, Pr denotes a propyl group and Bu denotes a butyl group.

The term "(C₃₋ₘ)cycloalkyl" as used herein, wherein m is an integer, either alone or in combination with another radical, means a cycloalkyl substituent containing from 3 to m carbon atoms and includes, but is not limited to, cyclopropyl, cyclobutyl, cyclopentyl, cyclohexyl and cycloheptyl.

The terms "O-(C₁₋ₙ)alkyl" or "(C₁₋ₙ)alkoxy" as used herein interchangeably, either alone or in combination with another radical, refer to an oxygen atom further bonded to an alkyl radical as defined above containing from 1 to n carbon atoms, and includes, but is not limited to, methoxy, ethoxy, propoxy, 1-methylethoxy, butoxy and 1,1-dimethylethoxy. The latter radical is known commonly as *tert*-butoxy. When an O-(C₁₋ₙ)alkyl radical is substituted, it is understood to be substituted on the (C₁₋ₙ)alkyl portion thereof.

As used herein, the terms "-S-(C₁₋ₙ)alkyl" or "(C₁₋ₙ)alkylthio" used interchangeably, refer to a sulfur atom further bonded to an alkyl radical as defined above containing from 1 to n carbon atoms. Examples of (C₁₋₆)alkylthio include, but are not limited to, methylthio (CH₃S-), ethylthio (CH₃CH₂S-), propylthio (CH₃CH₂CH₂S-), 1-methylethylthio (isopropylthio; (CH₃)₂CHS-), 1,1-dimethylethylthio (tert-butylthio; (CH₃)₃CS-), etc.. When an -S-(C₁₋ₙ)alkyl radical is substituted, it is understood to be substituted on the (C₁₋ₙ)alkyl portion thereof.

The term "halo" or "halogen" as used herein interchangeably means a halogen substituent selected from fluoro, chloro, bromo or iodo.

The term "salt thereof" means any acid and/or base addition salt of a compound according to the invention; preferably a pharmaceutically acceptable salt thereof.

The term "pharmaceutically acceptable salt" means a salt of a compound of formula (I) which is, within the scope of sound medical judgment, suitable for use in contact with the tissues of humans and lower animals without undue toxicity, irritation, allergic response, and the like, commensurate with a reasonable benefit/risk ratio, generally water or oil-soluble or dispersible, and effective for their intended use. The term includes pharmaceutically-acceptable acid addition salts and pharmaceutically-acceptable base addition salts. Lists of suitable salts are found in, e.g., S.M. Birge et al., J. Pharm. Sci., 1977, 66, pp. 1-19.

The term "pharmaceutically-acceptable acid addition salt" means those salts which retain the biological effectiveness and properties of the free bases and which are not biologically or otherwise undesirable, formed with inorganic acids such as hydrochloric acid, hydrobromic acid, sulfuric acid, sulfamic acid, nitric acid, phosphoric acid, and the like, and organic acids such as acetic acid, trifluoroacetic acid, adipic acid, ascorbic acid, aspartic acid, benzenesulfonic acid, benzoic acid, butyric acid, camphoric acid, camphorsulfonic acid, cinnamic acid, citric acid, digluconic acid, ethanesulfonic acid, glutamic acid, glycolic acid, glycerophosphoric acid, hemisulfic acid, hexanoic acid, formic acid, fumaric acid, 2-hydroxyethanesulfonic acid (isethionic acid), lactic acid, hydroxymaleic acid, malic acid, malonic acid, mandelic acid, mesitylenesulfonic acid, methanesulfonic acid, naphthalenesulfonic acid, nicotinic acid, 2-naphthalenesulfonic acid, oxalic acid, pamoic acid, pectinic acid, phenylacetic acid, 3-phenylpropionic acid, pivalic acid, propionic acid, pyruvic acid, salicylic acid, stearic acid, succinic acid, sulfanilic acid, tartaric acid, p-toluenesulfonic acid, undecanoic acid, and the like.

The term "pharmaceutically-acceptable base addition salt" means those salts which retain the biological effectiveness and properties of the free acids and which are not biologically or otherwise undesirable, formed with inorganic bases such as ammonia or hydroxide, carbonate, or bicarbonate of ammonium or a metal cation such as sodium, potassium, lithium, calcium, magnesium, iron, zinc, copper, manganese, aluminum, and the like. Particularly preferred are the ammonium, potassium, sodium, calcium, and magnesium salts. Salts derived from pharmaceutically-acceptable organic nontoxic bases include salts of primary, secondary, and tertiary amines, quaternary amine compounds, substituted amines including naturally occurring substituted amines, cyclic amines and basic ion-exchange resins, such as methylamine, dimethylamine, trimethylamine, ethylamine, diethylamine, triethylamine, isopropylamine, tripropylamine, tributylamine, ethanolamine, diethanolamine, 2-dimethylaminoethanol, 2-diethylaminoethanol, dicyclohexylamine, lysine, arginine, histidine, caffeine, hydrabamine, choline, betaine, ethylenediamine, glucosamine, methylglucamine, theobromine, purines, piperazine, piperidine, N-ethylpiperidine, tetramethylammonium compounds, tetraethylammonium compounds, pyridine, N,N-dimethylaniline, N-methylpiperidine, N-methylmorpholine, dicyclohexylamine, dibenzylamine, N,N-dibenzylphenethylamine, 1-ephenamine, N,N'-dibenzylethylenediamine, polyamine resins, and the like. Particularly preferred organic nontoxic bases are isopropylamine, diethylamine, ethanolamine, trimethylamine, dicyclohexylamine, choline, and caffeine.

The term "mammal" as it is used herein is meant to encompass humans, as well as non-human mammals which are susceptible to infection by hepatitis C virus. Non-human mammals include but are not limited to domestic animals, such as cows, pigs, horses, dogs and cats, and non-domestic animals.

The term "antiviral agent" as used herein means an agent (compound or biological) that is effective to inhibit the formation and/or replication of a virus in a mammal. This includes agents that interfere with either host or viral mechanisms necessary for the formation and/or replication of a virus in a mammal. Such agents can be selected from: an immunomodulatory agent, another anti-HCV agent (including but not limited to an inhibitor of HCV polymerase, another inhibitor of HCV NS3 protease or an inhibitor of another target in the HCV life cycle), an HIV inhibitor, an HAV inhibitor and an HBV inhibitor. Antiviral agents include, but are not limited to ribavirin, amantadine, VX-497 (merimepodib, Vertex Pharmaceuticals), Levovirin and Viramidine.

The term "immunomodulatory agent" as used herein means those agents (compounds or biologicals) that are effective to enhance or potentiate the immune system response in a mammal. Immunomodulatory agents include, for example, class I interferons (such as α-, β-, δ- and omega interferons, tau-interferons, consensus interferons and asialo-interferons), class 11 interferons (such as γ-interferons), pegylated interferons and conjugated interferons, including but not limited to interferons conjugated with other proteins including but not limited to human albumin.

The term "inhibitor of HCV NS3 protease" as used herein means an agent (compound or biological) that is effective to inhibit the function of HCV NS3 protease in a mammal. Inhibitors of HCV NS3 protease include, but are not limited to, those compounds described in WO 99/07733, WO 99/07734, WO 00/09558, WO 00/09543, WO 00/59929, WO 03/064416, WO 03/064455, WO 03/064456, WO 2004/037855, WO 2004/101602, WO 2004/101605, WO 2004/103996, WO 2005/028501, WO 2005/070955, WO 2006/000085, WO 2006/007700 and WO 2006/007708 (all by Boehringer Ingelheim), WO 02/060926, WO 03/053349, WO 03/099274, WO 03/099316, WO 2004/032827, WO 2004/043339, WO 2004/094452, WO 2005/046712, WO 2005/051410, WO 2005/054430 (all by BMS), WO 2004/072243, WO 2004/093798, WO 2004/113365, WO 2005/010029 (all by Enanta), WO 2005/037214 (Intermune) and WO 2005/051980 (Schering), and the candidates identified as VX-950 and SCH 503034.

The term "inhibitor of HCV polymerase" as used herein means an agent (compound or biological) that is effective to inhibit the function of an HCV polymerase in a mammal. This includes, but is not limited to, non-nucleoside and nucleoside inhibitors of HCV NS5B polymerase. Examples of inhibitors of HCV polymerase include but are not limited to those compounds described in: WO 02/04425, WO 03/007945, WO 03/010140, WO 03/010141, WO 2004/064925, WO 2004/065367, WO 2005/080388 and WO 2006/007693 (all by Boehringer Ingelheim), WO 2005/012288 (Genelabs), WO 2004/087714 (IRBM), WO 03/101993 (Neogenesis), WO 03/026587 (BMS), WO 03/000254 (Japan Tobacco), and WO 01/47883 (Japan Tobacco), and the clinical candidates HCV 796 (ViroPharma/Wyeth), R-1626 (Roche) and NM 283 (Idenix/Novartis).

The term "inhibitor of another target in the HCV life cycle" as used herein means an agent (compound or biological) that is effective to inhibit the formation and/or replication of HCV in a mammal other than by inhibiting the function of the HCV NS3 protease. This includes but is not limited to agents that interfere with either host or HCV viral mechanisms necessary for the formation and/or replication of HCV in a mammal. Inhibitors of another target in the HCV life cycle include, but are not limited to, agents that inhibit a target selected from a helicase, a NS2/3 protease and an internal ribosome entry site (IRES) and agents that interfere with the function of other viral targets including but not limited to an NS5A protein and an NS4B protein.

The term "HIV inhibitor" as used herein means an agent (compound or biological) that is effective to inhibit the formation and/or replication of HIV in a mammal. This includes but is not limited to agents that interfere with either host or viral mechanisms necessary for the formation and/or replication of HIV in a mammal. HIV inhibitors include, but are not limited to, nucleoside inhibitors, non-nucleoside inhibitors, protease inhibitors, fusion inhibitors and integrase inhibitors.

The term "HAV inhibitor" as used herein means an agent (compound or biological) that is effective to inhibit the formation and/or replication of HAV in a mammal. This includes but is not limited to agents that interfere with either host or viral mechanisms necessary for the formation and/or replication of HAV in a mammal. HAV inhibitors include but are not limited to Hepatitis A vaccines, for example, Havrix^{®} (GlaxoSmithKline), VAQTA^{®} (Merck) and Avaxim^{®} (Aventis Pasteur).

The term "HBV inhibitor" as used herein means an agent (compound or biological) that is effective to inhibit the formation and/or replication of HBV in a mammal. This includes but is not limited to agents that interfere with either host or viral mechanisms necessary for the formation and/or replication of HBV in a mammal. HBV inhibitors include, but are not limited to, agents that inhibit HBV viral DNA polymerase or HBV vaccines. Specific examples of HBV inhibitors include but are not limited to Lamivudine (Epivir-HBV^{®}), Adefovir Dipivoxil, Entecavir, FTC (Coviracil^{®}, DAPD (DXG), L-FMAU (Clevudine^{®}), AM365 (Amrad), Ldt (Telbivudine), monoval-LdC (Valtorcitabine), ACH-126,443 (L-Fd4C) (Achillion), MCC478 (Eli Lilly), Racivir (RCV), Fluoro-L and D nucleosides, Robustaflavone, ICN 2001-3 (ICN), Bam 205 (Novelos), XTL-001 (XTL), Imino-Sugars (Nonyl-DNJ) (Synergy), HepBzyme; and immunomodulator products such as: interferon alpha 2b, HE2000 (Hollis-Eden), Theradigm (Epimmune), EHT899 (Enzo Biochem), Thymosin alpha-1 (Zadaxin^{®}), HBV DNA vaccine (PowderJect), HBV DNA vaccine (Jefferon Center), HBV antigen (OraGen), BayHep B^{®} (Bayer), Nabi-HB^{®} (Nabi) and Anti-hepatitis B (Cangene); and HBV vaccine products such as the following: Engerix B, Recombivax HB, GenHevac B, Hepacare, Bio-Hep B, TwinRix, Comvax, Hexavac.

The term "class I interferon" as used herein means an interferon selected from a group of interferons that all bind to receptor type I. This includes both naturally and synthetically produced class I interferons. Examples of class I interferons include but are not limited to α-, β-, δ, ω- interferons, τ-interferons, consensus interferons, asialo-interferons and pegylated forms thereof.

The term "class II interferon" as used herein means an interferon selected from a group of interferons that all bind to receptor type II. Examples of class II interferons include but are not limited to γ-interferons.

Specific preferred examples of antiviral agents are listed below:
- ribavirin, amantadine and viramidine;
- immunomodulatory agents: class I interferons, class II interferons, pegylated interferons and conjugated interferons;
- HCV polymerase inhibitors: nucleoside analogs and non-nucleosides;
- inhibitor of another target in the HCV life cycle: agents that inhibit a target selected from a helicase, a NS2/3 protease and an internal ribosome entry site (IRES) and agents that interfere with the function of other viral targets including but not limited to an NS5A protein;
- HIV inhibitors: nucleoside inhibitors, non-nucleoside inhibitors, protease inhibitors, fusion inhibitors and integrase inhibitors; or
- HBV inhibitors: agents that inhibit viral DNA polymerase or is an HBV vaccine.

As discussed above, combination therapy is contemplated wherein a compound of formula (I), or a pharmaceutically acceptable salt thereof, is co-administered with at least one additional antiviral agent, such as an immunomodulatory agent, an inhibitor of HCV polymerase, another inhibitor of HCV NS3 protease, an inhibitor of another target in the HCV life cycle, an HIV inhibitor, an HAV inhibitor and an HBV inhibitor. Examples of such antiviral agents are provided in the Definitions section above. These additional agents may be combined with the compounds of this invention to create a single pharmaceutical dosage form. Alternatively these additional agents may be separately administered to the patient as part of a multiple dosage form, for example, using a kit. Such additional agents may be administered to the patient prior to, concurrently with, or following the administration of a compound of formula (I), or a pharmaceutically acceptable salt thereof.

As used herein, the term "treatment" means the administration of a compound or composition according to the present invention to alleviate or eliminate symptoms of the hepatitis C disease and/or to reduce viral load in a patient. The term "treatment" also encompasses the administration of a compound or composition according to the present invention post-exposure of the individual to the virus but before the appearance of symptoms of the disease, and/or prior to the detection of the virus in the blood, to prevent the appearance of symptoms of the disease and/or to prevent the virus from reaching detectable levels in the blood.

The following sign 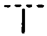 is used in sub-formulas to indicate the bond which is connected to the rest of the molecule as defined.

### Preferred embodiments

In the following preferred embodiments, groups and substituents of the compounds according to this invention are described in detail.

Included in the preferred embodiments of the invention are compounds of formula I wherein:

### R¹:

Preferably, **R¹** is selected from ethenyl and ethyl.
Any and each individual definition of **R¹** as set out herein may be combined with any and each individual definition of **R², R³, R⁴** and **R⁶** as set out herein.

### R²;

In one preferred embodiment **R²** is a group of formula: 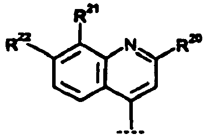 wherein **R²⁰, R²¹** and **R²²** are as defined herein.

Preferably, **R²⁰** is methoxy, ethoxy, propoxy, 1-methylethoxy, methylthio, ethylthio, propylthio or 1-methylethylthio.

More preferably, **R²⁰** is methoxy, ethoxy, methylthio or ethylthio.

Most preferably, **R²⁰** is methoxy, ethoxy or ethylthio.

Preferably, **A²¹** is H, fluoro, chloro, bromo, methyl, ethyl, methoxy, ethoxy, methylthio or ethylthio.

More preferably, **R²¹** is H, chloro, bromo, methyl, methoxy or methylthio.

Preferably, **R²²** is H, methoxy or ethoxy.

Most preferably, **R²²** is H or methoxy.

Therefore preferably, **R²** is selected from the following formulas: 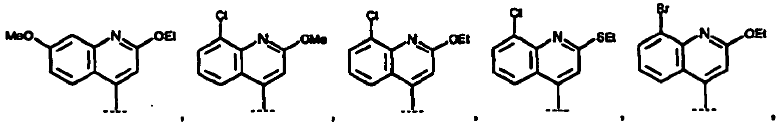 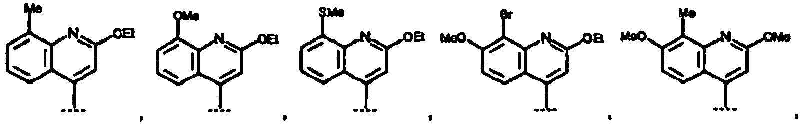 and

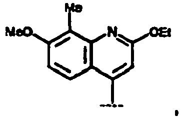

Any and each individual definition of **R²** (including any and each combination of individual definitions of **R²⁰**, **R²¹, R²², R²⁴** and **R²⁵**) as set out herein may be combined with any and each individual definition of **R¹, R³, R⁴** and **R⁶** as set out herein.

### R³:

Most preferably, **R³** is 1,1-dimethylethyl.

Any and each individual definition of **R³** as set out herein may be combined with any and each individual definition of **R¹, R², R⁴** and **R⁵** as set out herein.

### R⁴:

Preferably, **R⁴** is cyclopropyl or cyclobutyl, each of which being optionally substituted with methyl, ethyl, propyl or 1-methylethyl.

More preferably, **R⁴** is cyclopropyl or cyclobutyl, each of which being optionally substituted at the 1-position with methyl, ethyl, propyl or 1-methylethyl.

Even more preferably, **R⁴** is cyclopropyl, cyclobutyl, 1-methylcyclopropyl or 1-methylcyclobutyl.

Most preferably, **R⁴** is cyclopropyl or 1-methylcyclopropyl.

Any and each individual definition of **R⁴** as set out herein may be combined with any and each individual definition of **R¹, R², R³** and **R⁵** as set out herein.

### R⁵:

In one preferred embodiment, **R⁶** is **R⁵⁰**-O-, wherein **R⁵⁰** is as defined herein.

In an alternative preferred embodiment, **R⁵** is **R⁵⁰**-NH-; wherein **R⁵⁰** is as defined herein.

Preferably, **R⁵⁰** is selected from ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 1,1-dimethylpropyl, 1,2-dimethylpropyl, 2,2-dimethylpropyl, 1-ethylpropyl, cyclopropyl, cyclobutyl, cyclopentyl and cyclohexyl, the cyclopropyl, cyclobutyl, cyclopentyl and cyclohexyl each being optionally substituted with methyl or ethyl. Most preferably, **R⁵⁰** is selected from 1,1-dimethylethyl and cyclopentyl.

Therefore, most preferably, **R⁶** is **R⁵⁰**-O-, wherein **R⁵⁰** is selected from 1,1-dimethylethyl and cyclopentyl.

Any and each individual definition of **R⁵** as set out herein may be combined with any and each individual definition of **R¹**, **R²**, **R³** and **R⁴** as set out herein.

A preferred embodiment provides compounds of formula (I) wherein
- **R¹**: is selected from ethenyl and ethyl;
**R²** is a group of formula 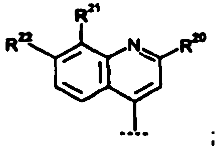 wherein
- **R³**: **R²⁰** is methoxy, ethoxy, propoxy, 1-methylethoxy, methylthio, ethylthio, propylthio or 1-methylethylthio; **R²¹** is H, fluoro, chloro, bromo, methyl, ethyl, methoxy, ethoxy, methylthio or ethylthio; **R²²** is H, methoxy or ethoxy; is 1,1-dimethylethyl; and
- **R⁴**: is cyclopropyl or cyclobutyl, each of which being optionally substituted with methyl, ethyl, propyl or 1-methylethyl;
- **R⁵**: is **R⁵⁰**-O- or **R⁵⁰**-NH-; wherein **R⁵⁰** is selected from ethyl, propyl, 1-methylethyl, butyl, 1-methylpropyl, 2-methylpropyl, 1,1-dimethylethyl, pentyl, 1-methylbutyl, 2-methylbutyl, 3-methylbutyl, 1,1-dimethylpropyl, 1,2-dimethylpropyl, 2,2-dimethylpropyl, 1-ethylpropyl, cyclopropyl, cyclobutyl, cyclopentyl and cyclohexyl, the cyclopropyl, cyclobutyl, cyclopentyl and cyclohexyl each being optionally substituted with methyl or ethyl.

More preferably in this embodiment:
- **R¹**: is selected from ethenyl and ethyl;
- **R²**: is a group of formula 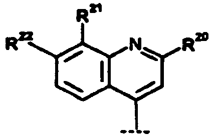 wherein
**R²⁰** is methoxy, ethoxy or ethylthio;
**R²¹** is H, chloro, bromo, methyl, methoxy or methylthio;
**R²²**
- **R³**: is 1,1-dimethylethyl; and
- **R⁴**: is cyclopropyl or 1-methylcyclopropyl;
- **R⁵**: is **R⁵**-O-; wherein **R⁵⁰** is selected from 1,1-dimethylethyl and cyclopentyl.

### Specific examples of preferred embodiments:

Examples of most preferred compounds according to this invention are each single compound listed in the following Table 1.

In general, all tautomeric and isomeric forms and mixtures thereof, for example, individual geometric isomers, stereoisomers, enantiomers, diastereomers, racemates, racemic or non-racemic mixtures of stereoisomers, mixtures of diastereomers, or mixtures of any of the foregoing forms of a chemical structure or compound is intended, unless the specific stereochemistry or isomeric form is specifically indicated in the compound name or structure.

It is well-known in the art that the biological and pharmacological activity of a compound is sensitive to the stereochemistry of the compound. Thus, for example, enantiomers often exhibit strikingly different biological activity including differences in pharmacokinetic properties, including metabolism, protein binding, and the like, and pharmacological properties, including the type of activity displayed, the degree of activity, toxicity, and the like. Thus, one skilled in the art will appreciate that one enantiomer may be more active or may exhibit beneficial effects when enriched relative to the other enantiomer or when separated from the other enantiomer. Additionally, one skilled in the art would know how to separate, enrich, or selectively prepare the enantiomers of the compounds of the present invention from this disclosure and the knowledge in the art.

Preparation of pure stereoisomers, e.g. enantiomers and diastereomers, or mixtures of desired enantiomeric excess (ee) or enantiomeric purity, are accomplished by one or more of the many methods of (a) separation or resolution of enantiomers, or (b) enantioselective synthesis known to those of skill in the art, or a combination thereof. These resolution methods generally rely on chiral recognition and include, for example, chromatography using chiral stationary phases, enantioselective host-guest complexation, resolution or synthesis using chiral auxiliaries, enantioselective synthesis, enzymatic and nonenzymatic kinetic resolution, or spontaneous enantioselective crystallization. Such methods are disclosed generally in Chiral Separation Techniques: A Practical Approach (2nd Ed.), G. Subramanian (ed.), Wiley-VCH, 2000; T.E. Beesley and R.P.W. Scott, Chiral Chromatography, John Wiley & Sons, 1999; and Satinder Ahuja, Chiral Separations by Chromatography, Am. Chem. Soc., 2000. Furthermore, there are equally well-known methods for the quantitation of enantiomeric excess or purity, for example, GC, HPLC, CE, or NMR, and assignment of absolute configuration and conformation, for example, CD ORD, X-ray crystallography, or NMR.

### Pharmaceutical composition:

According to an alternate embodiment, the pharmaceutical composition of this invention may additionally comprise at least one other anti-HCV agent. Examples of anti-HCV agents include, α- (alpha), β- (beta), δ- (delta), γ- (gamma), ω- (omega) or τ-(tau) interferon, pegylated α-interferon, ribavirin and amantadine.

According to another alternate embodiment, the pharmaceutical composition of this invention may additionally comprise at least one other inhibitor of HCV NS3 protease.

According to another alternate embodiment, the pharmaceutical composition of this invention may additionally comprise at least one inhibitor of HCV polymerase.

According to yet another alternate embodiment, the pharmaceutical composition of this invention may additionally comprise at least one inhibitor of other targets in the HCV life cycle, including but not limited to agents that inhibit a target selected from a helicase, a NS2/3 protease and an internal ribosome entry site (IRES) and agents that interfere with the function of other viral targets including but not limited to an NS5A protein.

The pharmaceutical composition of this invention may be administered orally, parenterally or via an implanted reservoir. Oral administration or administration by injection is preferred. The pharmaceutical composition of this invention may contain any conventional non-toxic pharmaceutically-acceptable carriers, adjuvants or vehicles. In some cases, the pH of the formulation may be adjusted with pharmaceutically acceptable acids, bases or buffers to enhance the stability of the formulated compound or its delivery form. The term parenteral as used herein includes subcutaneous, intracutaneous, intravenous, intramuscular, intra-articular, intrasynovial, intrasternal, intrathecal, and intralesional injection or infusion techniques.

The pharmaceutical composition may be in the form of a sterile injectable preparation, for example, as a sterile injectable aqueous or oleaginous suspension. This suspension may be formulated according to techniques known in the art using suitable dispersing or wetting agents (such as, for example Tween 80) and suspending agents.

The pharmaceutical composition of this invention may be orally administered in any orally acceptable dosage form including, but not limited to, capsules, tablets, and aqueous suspensions and solutions. In the case of tablets for oral use, carriers which are commonly used include lactose and corn starch. Lubricating agents, such as magnesium stearate, are also typically added. For oral administration in a capsule form, useful diluents include lactose and dried corn starch. When aqueous suspensions are administered orally, the active ingredient is combined with emulsifying and suspending agents. If desired, certain sweetening and/or flavoring and/or coloring agents may be added.

Other suitable vehicles or carriers for the above noted formulations and compositions can be found in standard pharmaceutical texts, e.g. in "Remington's Pharmaceutical Sciences", The Science and Practice of Pharmacy, 19th Ed. Mack Publishing Company, Easton, Penn., (1995).

Dosage levels of between about 0.001 and about 100 mg/kg body weight per day, preferably between about 0.01 and about 50 mg/kg body weight per day of the protease inhibitor compound described herein are useful in a monotherapy for the treatment of HCV mediated disease. Typically, the pharmaceutical composition of this invention will be administered from about 1 to about 5 times per day or alternatively, as a continuous infusion. Such administration can be used as a chronic or acute therapy. The amount of active ingredient that may be combined with the carrier materials to produce a single dosage form will vary depending upon the host treated and the particular mode of administration. A typical preparation will contain from about 5% to about 95% active compound (*w*/*w*). Preferably, such preparations contain from about 20% to about 80% active compound.

As the skilled artisan will appreciate, lower or higher doses than those recited above may be required. Specific dosage and treatment regimens for any particular patient will depend upon a variety of factors, including the activity of the specific compound employed, the age, body weight, general health status, sex, diet, time of administration, rate of excretion, drug combination, the severity and course of the infection, the patient's disposition to the infection and the judgment of the treating physician. Generally, treatment is initiated with small dosages substantially less than the optimum dose of the peptide. Thereafter, the dosage is increased by small increments until the optimum effect under the circumstances is reached. In general, the compound is most desirably administered at a concentration level that will generally afford antivirally effective results without causing any harmful or deleterious side effects.

When the composition of this invention comprises a combination of a compound of formula I and one or more additional therapeutic or prophylactic agent, both the compound and the additional agent should be present at dosage levels of between about 10 to 100%, and more preferably between about 10 and 80% of the dosage normally administered in a monotherapy regimen.

When these compounds, including their pharmaceutically acceptable salts and esters thereof, are formulated together with a pharmaceutically acceptable carrier, the resulting composition may be administered *in vivo* to mammals, such as man, to inhibit HCV NS3 protease or to treat HCV virus infection. Such treatment may also be achieved using a compound of this invention in combination with another antiviral agent. Preferred other antiviral agents are described within the Definitions section and the section of preferred pharmaceutical compositions according to this invention and include, but are not limited to: α- (alpha), β- (beta), δ- (delta), ω- (omega), γ-(gamma) or τ- (tau) -interferon, ribavirin, amantadine; other inhibitors of HCV NS3 protease; inhibitors of HCV polymerase; inhibitors of other targets in the HCV life cycle, which include but are not limited to, agents that inhibit a target selected from a helicase, a NS2/3 protease and an internal ribosome entry site (IRES) and agents that interfere with the function of other viral targets including but not limited to an NS5A protein; or combinations thereof. The additional agents may be combined with compounds of this invention to create a single dosage form. Alternatively these additional agents may be separately administered to a mammal as part of a multiple dosage form.

As discussed above, combination therapy is contemplated wherein a compound of formula (I), or a pharmaceutically acceptable salt or ester thereof, is co-administered with at least one additional antiviral agent Preferred antiviral agents are described hereinbefore and examples of such agents are provided in the Definitions section. These additional agents may be combined with the compounds of this invention to create a single pharmaceutical dosage form. Alternatively these additional agents may be separately administered to the patient as part of a multiple dosage form, for example, using a kit. Such additional agents may be administered to the patient prior to, concurrently with, or following the administration of a compound of formula (I), or a pharmaceutically acceptable salt or ester thereof.

A compound of formula (I), or a pharmaceutically acceptable salt or ester thereof, set forth herein may also be used as a laboratory reagent. Furthermore a compound of this invention, including a pharmaceutically acceptable salt or ester thereof, may also be used to treat or prevent viral contamination of materials and therefore reduce the risk of viral infection of laboratory or medical personnel or patients who come in contact with such materials (e.g. blood, tissue, surgical instruments and garments, laboratory instruments and garments, and blood collection apparatuses and materials).

A compound of formula (I), including a pharmaceutically acceptable salt or ester thereof, set forth herein may also be used as a research reagent. A compound of formula (I), including a pharmaceutically acceptable salt or ester thereof, may also be used as positive control to validate surrogate cell-based assays or *in vitro* or *in vivo* viral replication assays.

A process for the preparation of compounds of formula (I) comprises:
a) reacting a compound of formula (III) 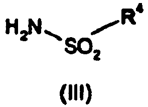 wherein R⁴ is as defined herein, with a strong base so as to form the corresponding amide anion of formula (IIIa) 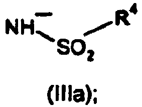 and
b) reacting an azalactone of formula (II): 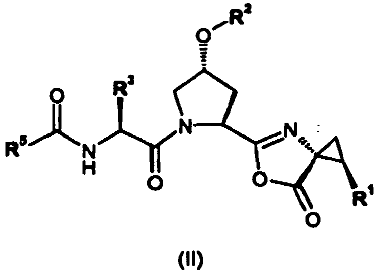
wherein R¹, R², R³ and R⁵ are as defined herein, with the amide anion of formula IIIa. The strong base referred to in step a) is well known to one skilled in the art and includes, but is not limited to, an alkyllithium reagent (including, but not limited to, butyllithium, *tert*-butyllithium and the like) and the alkali metal salt of a secondary amine or silyl analog thereof (including, but not limited to, lithium hexamethyldisilazide, sodium hexamethyldisilazide, potassium hexamethyldisilazide, lithium diisopropylamide, lithium N-isopropylcyclohexylamide, lithium tetramethylpiperidide, potassium diisopropylamide, and the like).

### METHODOLOGY

The compounds of the present invention are synthesized according to a general process wherein the P3, P2, P1, and P1' fragments can be linked by well known peptide coupling techniques. The P3, P2, P1, and P1' fragments may be linked together in any order as long as the final compound corresponds to compounds of formula (I), wherein R¹, R², R³, R⁴, and R⁵ are as defined herein. For example, P3 can be linked to P2-P1-P1', or P1-P1' linked to P3-P2. This process is illustrated in Scheme I (wherein CPG is a carboxyl protecting group and APG is an amino protecting group).

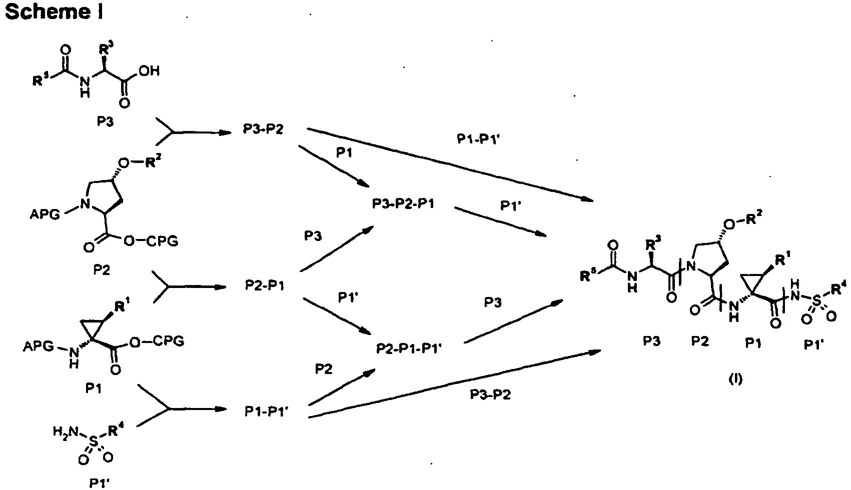

Generally, the P3, P2 and P1 fragments are coupled by deprotecting the α-amino group of the N-terminal residue and coupling the unprotected carboxyl group of the next suitably N-protected amino acid through a peptide linkage using well known methods, such as those described in general textbooks on peptide chemistry, for example, M. Bodanszky, "Peptide Chemistry", 2nd rev ed., Springer-Verlag, Berlin, Germany, (1993). This deprotection and coupling procedure is repeated until the desired sequence is obtained. This coupling can be performed with the constituent amino acid fragments in stepwise fashion or by solid phase peptide synthesis according to the method originally described in Merrifield, J. Am. Chem. Soc., (1963), 85, 2149-2154. Other methods of peptide synthesis are described in Stewart and Young, "Solid Phase Peptide Synthesis", 2nd ed., Pierce Chemical Co., Rockford, IL (1984); Gross, Meienhofer, Udenfriend, Eds., "The Peptides: Analysis, Synthesis, Biology", Vol. 1, 2, 3, 5, and 9, Academic Press, New-York, (1980-1987); Bodansky et al., "The Practice of Peptide Synthesis" Springer-Verlag, New-York (1984).

The P1' fragments R⁴-S(O)ₘNH₂ are coupled to the P1, P2-P1 or P3-P2-P1 fragments in the presence of a coupling agent under standard conditions. Although several commonly used coupling agents can be employed, TBTU and HATU have been found to be practical. Alternatively, an azalactone of formula (II): 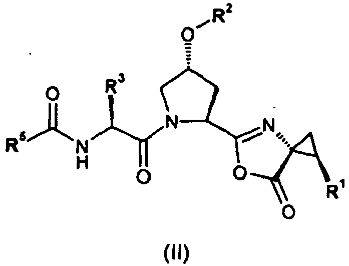 may be treated by the amide anion (IIIa): 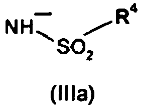 as described hereinabove, to effect the coupling reaction and prepare compounds of formula (I). The azalactone is readily prepared from the precursor carboxylic acid by treatment with a dehydrating agent such as isobutylchloroformate or the like, as shown in Scheme II below.

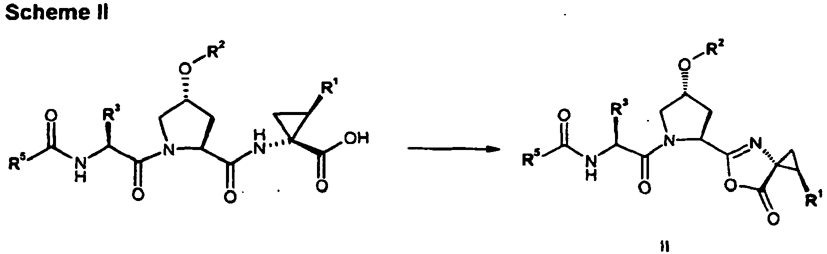

### Synthesis of P1' Fragments

P1' fragments of formula R⁴SO₂NH₂ are available commercially or are prepared by known methods or by procedures described in the following examples.

### Synthesis of P1 Fragments

P1 moieties of compounds of formula (I) are prepared using the protocols outlined in WO 00/59929 and WO 00/09543. In particular, reference is made to pages 33-35, Example 1 of WO00/59929 and Pages 56-69, Example 9 - 20 of WO00/09543 for the preparation of 1-aminocyclopropanecarboxylic acid P1 moieties.

### Synthesis of P2 Fragments

A suitable **R²** moiety may be attached to the P2 fragments using general methodology described in WO 2004/103996. Briefly, an appropriately protected cis-hydroxyproline fragment is obtained from the readily available *trans*-hydroxyproline derivative by Mitsunobu reaction with *para*-nitrobenzoic acid, followed by hydrolysis of the *para-*nitrobenzoate ester. The cis-hydroxyproline fragment is then reacted, with inversion of stereochemistry, with a hydroxylated **R²** moiety (quinoline or thienopyridine) by using either a Mitsunobu reaction or by converting the free hydroxyl group into a good leaving group (such as a brosylate) and displacing it with the hydroxylated **R²** moiety. The general methodology is illustrated in Scheme III below.

This attachment may take place at any stage in the process illustrated in Scheme I, i.e., when P2 is an isolated, suitably protected fragment or when it has already been coupled to P3 and/or P1 or P1-P1'. In cases where the **R²** moiety is to be added at an intermediate stage after coupling to the P3 and/or P1 or P1-P1' fragments, the P2 fragment shown above is replaced with a suitable precursor fragment for the purposes of this scheme.

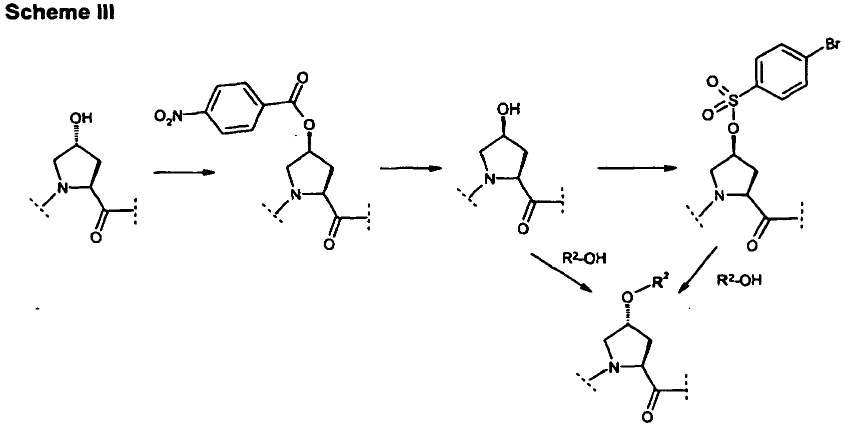

The compounds of formula **R²**-OH used as starting material may be synthesized from commercially available materials using techniques described in the literature. General methodology is illustrated in the following schemes.

Compounds of formula **R²**-OH wherein **R²** is a group of formula 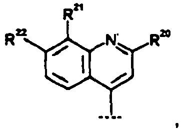 wherein **R²⁰** is -O-(C₁₋₆)alkyl, can be prepared according to the following scheme IV:

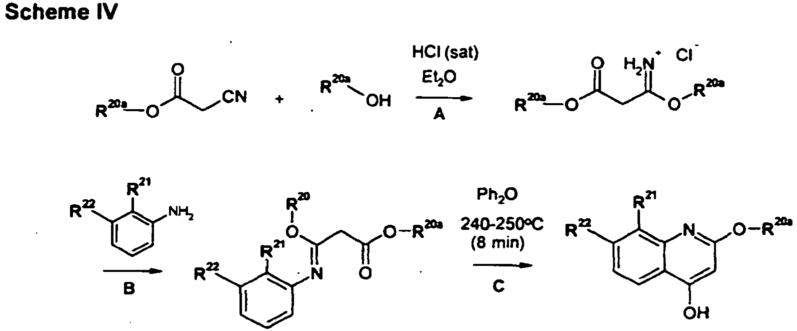

Briefly, following the known Pinner synthesis, a suitably functionalized cyanoester is condensed with the corresponding alcohol using a fully saturated HCl/Et₂O solution [Neilson, in Patai, "The Chemistry of Amidines and Imidates." pp. 385-489, Wiley, NY, 1975.]. The resulting imidate salt is then subsequently condensed with an appropriately substituted aniline to form the aniline derived imidate. Thermal cyclization affords the corresponding 2-alkoxy substituted 4-hydroxyquinolines.

For example, when **R^{20a}** = Et in the above scheme, ethyl cyanoacetate and ethanol are used as reagents. For **R^{20a}** = Me in the above scheme, methyl cyanoacetate and methanol are used as reagents.

The corresponding compounds of formula **R²**-OH wherein **R²⁰** is -S-(C₁₋₆)alkyl are prepared as shown in the following scheme V.

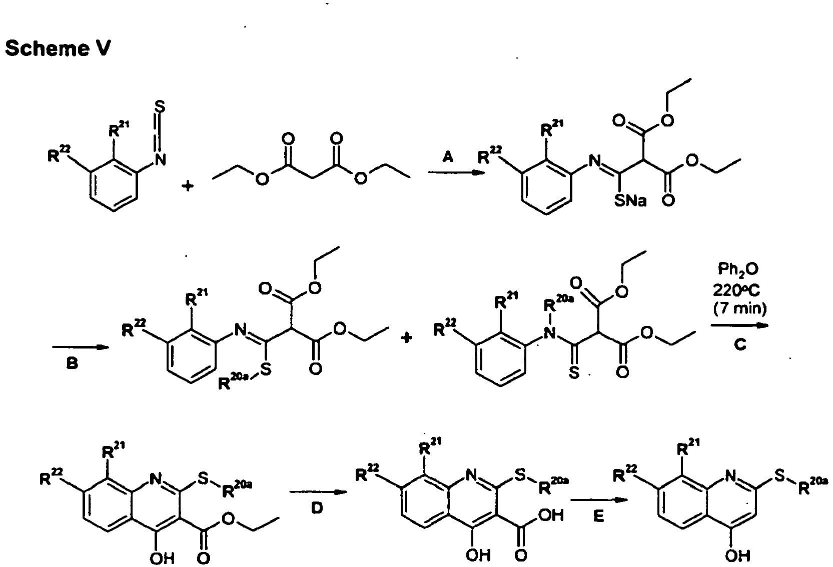

Briefly, condensation of diethyl malonate under basic conditions with a suitably functionalized isothiocyanate produces the malonate adduct as a salt. Treatment of the salt with an alkylating reagent (e.g. Etl: ethyl iodide) produces a mixture of S- and N-alkylated products. Thermal cyclization of this mixture gives the 3-ethyl carboxylate which is saponified and decarboxylated to produce the desired 2-thioalkyl substituted hydroxyquinolines. For example, utilization of Etl in the alkylation step results in the formation of the 2-thioethyl analog (**R^{20a}** = Et).

The corresponding anilines are commercially available or may be available from commercially available precursors using well known chemical transformations. For example if the nitro analog is commercially available, it can be converted to the corresponding amine by using any of several reducing agents well known to one skilled in the art. Alternatively, if the carboxylic acid is commercially available, transformation into the corresponding amine is possible via a Curtius rearrangement.

### Synthesis of P3 Fragments

The P3 carbamate fragments wherein **R⁵** is **B**-O-C(=O)- are prepared as described in WO 03/064416. The preparation of analogous P3 urea fragments wherein **R⁵** is **B**-NH-C(=O)- is described in WO 03/064456.

Further details of the invention are illustrated in the following examples which are understood to be non-limiting with respect to the appended claims. Other specific ways of synthesis or resolution of the compounds of this invention can be found in WO 00/09543, WO 00/09558, WO 00/59929, WO 03/064456 and WO 2004/103996.

### EXAMPLES

Temperatures are given in degrees Celsius. Solution percentages express a weight to volume relationship, and solution ratios express a volume to volume relationship, unless stated otherwise. Nuclear magnetic resonance (NMR) spectra were recorded on a Bruker 400 MHz spectrometer; the chemical shifts (δ) are reported in parts per million. Flash chromatography was carried out on silica gel (SiO₂) according to W.C. Still *et al.,* J. Org. Chem., (1978), 43, 2923. Analytical HPLC was carried out under standard conditions using a Combiscreen ODS-AQ C18 reverse phase column, YMC, 50 x 4.6 mm i.d., 5 µM, 120 A at 220 nM, elution with a linear gradient as described in the following table (Solvent A is 0.06% TFA in H₂O; solvent B is 0.06% TFA in CH₃CN):

| Time (min) | Flow (mL/min) | Solvent A (%) | Solvent B (%) |
|---|---|---|---|
| 0 | 3.0 | 95 | 5 |
| 0.5 | 3.0 | 95 | 5 |
| 6.0 | 3.0 | 50 | 50 |
| 10.5 | 3.5 | 0 | 100 |

### Abbreviations used in the examples include:

DBU: 1,8-diazabicyclo[5.4.0]undec-7-ene;
DCM: dichloromethane;
DIAD: diisopropylazodicarboxylate;
DIEA: diisopropylethylamine;
DI PEA: diisopropylethyl amine;
DMF: N, *N*-dimethylformamide;
DMAP: 4-(dimethylamino) pyridine;
EtOAc: ethyl acetate;
HATU: [O-7-azabenzotriazol-1-yl)-1,1,3,3-tetramethyluronium hexafluorophosphate];
HPLC: high performance liquid chromatography;
MS: mass spectrometry (MALDI-TOF: Matrix Assisted Laser Desorption Ionization-Time of Flight, FAB: Fast Atom Bombardment);
Me: methyl;
MeOH: methanol;
Ph: phenyl;
R.T.: room temperature (18°C to 22°C);
*tert*-butyl or t-butyl: 1,1-dimethylethyl;
Tbg: *tert-*butyl glycine: *tert*-leucine;
TBTU: 2-(1H-benzotriazole-1-yl)-1,1,3,3-tetramethyl uronium tetrafluoroborate;
TFA: trifluoroacetic acid; and
THF: tetrahydrofuran.

### EXAMPLE 1 A

### Synthesis of P3 carbamate fragment 1A1:

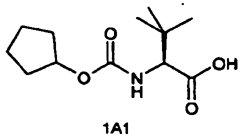

The P3 carbamate fragment **1A1** was prepared as described in WO 03/064416 and WO 03/064456.

It will be apparent to one skilled in the art that analogous P3 carbamate fragments in which the cyclopentyl group has been replaced by another **R⁵⁰** substituent as defined herein and/or the *tert*-butyl group has been replaced by another **R³** substituent as defined herein may be prepared using an analogous procedure.

### EXAMPLE 1B

### Synthesis of P3 urea fragment 1B1:

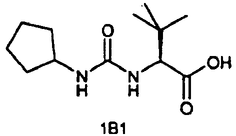

The preparation of P3 urea fragment **1B1** is described in WO 03/064456. Such fragments may be readily substituted for the P3 carbamate fragments in the examples below, to provide compounds of formula (I) wherein **R⁵** is **R⁵⁰**-NH- and **R⁵⁰** is cyclopentyl.

It will be apparent to one skilled in the art that analogous P3 urea fragments in which the cyclopentyl group has been replaced by another **R⁵⁰** substituent as defined herein and/or the *tert*-butyl group has been replaced by another **R³** substituent as defined herein may be prepared using an analogous procedure.

### EXAMPLE 2A

### Synthesis of 1-methyl-2-methoxy aniline (2A2)

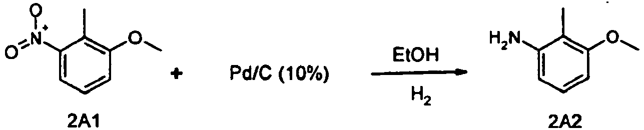

To a solution of 2-methyl-3-nitro anisole (**2A1**) (5.1 g ; 30.33 mmol ; requires -30min. to dissolve) in absolute ethanol (85 mL) was added 10% Pd/C catalyst (500 mg) . The solution was hydrogenated under a hydrogen filled balloon at atmospheric pressure and room temperature for 19 h. The reaction mixture was filtered through a Celite pad, rinsed and evaporated to dryness to obtain the compound **2A2** as a deep mauve oil (4.1 g ; 29.81 mmol ; 98% yield).

MS 137 (MH)+. Reverse Phase HPLC Homogeneity @ 220nm (0.06 % TFA ; CH₃CN : H₂O): 99%.

### EXAMPLE 2B

### Synthesis of 2-bromo-3-methoxy aniline (2B4)

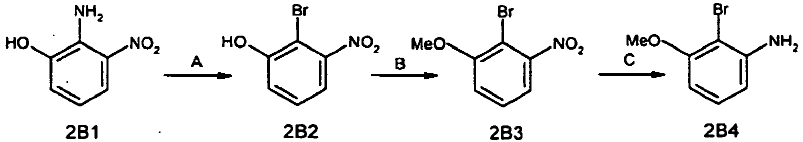

### Step A:

2-Amino-3-nitrophenol **2B1** (5 g; 32.4 mmol) was dissolved in H₂O (29.5 mL) and 1,4-dioxane (14.7 mL). The mixture was heated to reflux and hydrobromic acid (48%; 16.7 mL; 147 mmol) was added dropwise over a period of 20 min. Upon completion of the addition, the reflux was maintained an additional 15 min. The reaction was cooled to 0°C (ice bath), and sodium nitrite (2.23 g; 32.3 mmol) in H₂O (20 mL) was added over a period of 30 min. The stirring was continued for 15 min. at 0°C, the mixture transferred to a jacketed dropping funnel (0°C) and added dropwise to a stirred mixture of Cu(I)Br (5.34 g; 37.2 mmol) in H₂O (29.5 mL) and HBr (48%; 16.7 mL; 147 mmol) at 0°C. The reaction was stirred for 15 min. at 0°C, warmed to 60°C, stirred for an additional 15 min., cooled to room temperature, and left to stir overnight. The reaction mixture was transferred to a separatory funnel and extracted with ether (3 X 150 mL). The organic layers were combined, washed with brine (1 X), dried (Na₂SO₄), filtered and concentrated to afford the crude product (7.99 g) as a red-brown oil. The crude material was purified by flash column chromatography (1:25 ultra pure silica gel, 230-400 mesh, 40-60mm, 60 angstroms; CH₂Cl₂ as the solvent) to afford pure 2-bromo-3-nitro-phenol **2B2** (45%; 3.16 g) as an orange-brown solid.
MS 217.8 (MH)⁻. Homogeneity by HPLC (TFA) @ 220 nm: 97%.

### Step B:

The nitrophenol starting material **2B2** (3.1 g; 14.2 mmol) was dissolved in DMF (20 mL) and to the solution was added ground cesium carbonate (5.58 g; 17.1 mmol) followed by Mel (2.6 mL; 42.5 mmol). The mixture was stirred at room temperature overnight. The DMF was evaporated, the residue taken up in ether (1 X 200 mL), washed with water (1 X 200 mL), brine (4 X 100 mL), dried (MgSO₄), filtered and evaporated to afford the crude 2-bromo-3-nitroanisole **2B3** (94%; 3.1 g) as an orange solid.
MS 234 (M+2H)⁺; Homogeneity by HPLC (TFA) @ 220nm: 98%

### Step C:

2-Bromo-3-nitroanisole **2B3** (1.00 g; 4.31 mmol) was dissolved in glacial acetic acid (11.0 mL)/ethanol (11.0 mL) and to the solution was added iron powder (0.98 g; 17.5 mmol). The mixture was stirred at reflux for 3.5 h and worked up. The reaction mixture was diluted with water (35 mL), neutralized with solid Na₂CO₃ and the product extracted with CH₂Cl₂ (3X 50 mL). The extracts were dried (Na₂SO₄), filtered and concentrated in vacuo to afford the crude product, 2-bromo-3 methoxyaniline **2B4** (91%; 0.79 g) as a pale yellow oil.
MS 201.8 (MH)⁺; Homogeneity by HPLC (TFA) @ 220nm: 95%

### EXAMPLE 3A

### Preparation of 2-ethoxy-4-hydroxy-8-chloroquinoline (3A5)

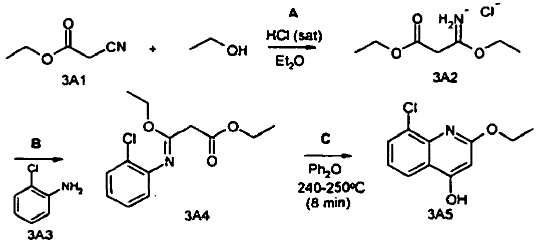

### Step A:

To ethyl cyanoacetate **3A1** (23 g, 0.203 mol) was added absolute ethanol (10 g, 12.7 mL, 0.22 mol) in diethyl ether (20 mL). The solution was cooled to 0°C in an ice bath before being treated with HCl gas (bubbled through solution for 12 minutes resulted in an increase in weight of 12 g (-0.33 mol)). This solution was stirred at 0°C for 6 h, allowed to warm to R.T. and stirred for 16 h. The resultant solid was broken up and washed several times with ether and then placed in *vacuo* for several hours. The imidate salt **3A2** was obtained as a white solid (36.4 g, 92%) and was stored under a nitrogen atmosphere. The ¹H NMR is consistent with the desired product.

### Step B:

The imidate salt **3A2** (1.47 g, 7.5 mmol, 1 eq.) was combined with 2-chloroaniline **3A3** (0.96 g, 7.50 mmol, 1 eq.) in ethanol (15 mL) under an N₂ atmosphere. The reaction mixture was stirred at R.T. (16 h) and monitored by HPLC. The reaction mixture was concentrated and then purified directly over silica gel (eluent: 10% EtOAc/hexanes) to afford the condensation product **3A4** as a clear oil (1.73 g, 86%). MS electrospray: (MH)⁺: 270 and (M - H)⁻; 268. TLC (UV) R_{f} = 0.50 (10% EtOAc/hexane).

### Step C:

The condensation product **3A4** (1.73 g, 6.41 mmol) was dissolved in diphenyl ether (10 mL) and placed in a sand bath (300°C). The internal temperature was monitored and maintained between 240-250°C for 8 minutes. The mixture was cooled and then directly loaded on a silica gel column and eluted first with hexanes, then with 30% EtOAc/hexanes and finally 50% EtOAc/hexanes. The product was concentrated and dried in *vacuo* to give the corresponding 4-hydroxyquinoline derivative **3A5** as a beige crystalline solid (0.76 g, 53%). MS electrospray: (M + H)⁺; 224 and (M - H)⁻; 222.

### EXAMPLE 3B

### Preparation of 2-thioethyl-8-chloro-4-hydroxyquinoline (3B7):

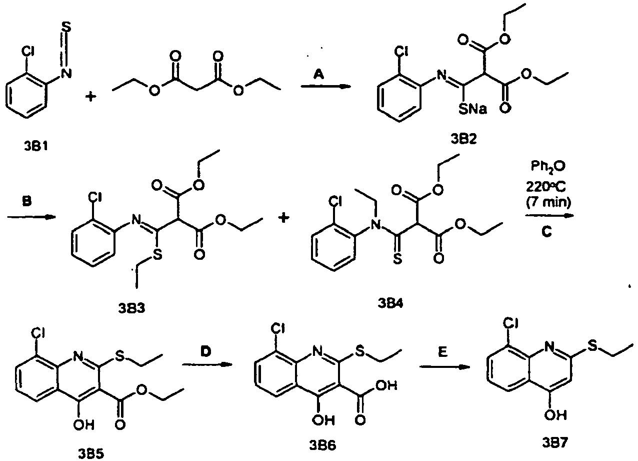

### Step A:

To THF (30 mL) was added sodium hydride (60% in oil, 920 mg, 23 mmol, 1.2 eq) before being cooled to 0°C. Diethyl malonate (2.91 mL, 19.15 mmol, 1.0 eq) was then added dropwise (gas evolution) and this solution was allowed to warm to R.T. and was stirred for 1 h. This mixture was cooled down to 0°C before the addition of 2-chlorophenyl isothiocyanate **3B1** (2.5 mL, 19.15 mmol, 1.0 eq). The resulting mixture was again allowed to warm to R.T. for 3 h until the starting material was consumed. The orange solution was concentrated down and dried in *vacuo* to afford the sodium salt adduct **3B2** (6.73 g, 100%) as an orange crystalline solid. This material was used as is in the following step B.

### Step B:

A solution of adduct **3B2** (6.0 g, 17.06 mmol, 1 eq) in DMF (50 mL) was cooled to -45°C. Ethyl iodide (1.64 mL, 20.5 mmol, 1.2 eq) was then slowly added and the solution was stirred at -45°C for 2 h and then at R.T. (16 h). Water was added and the mixture was extracted twice with a mixture of ether/hexanes (1:1, 3 X 150 mL). The combined organic fractions were washed with water (2x), dried over MgSO₄, filtered and concentrated to afford approximately a 1:1 mixture of **3B3** and **3B4** (S versus N alkylation) (6.1 g, 100%) as a yellow oil. This mixture was used in the following step since only the S-alkylated analog cyclizes.

### Step C:

In a pre-heated sand bath (350°C) a solution of compounds **3B3** and **3B4** (6.1 g, 17.05 mmol, 1 eq.) in diphenyl ether (60 mL) was heated until the internal temperature reached 220°C, which was maintained for 7 minutes. The solution was cooled to R.T. and the mixture loaded directly on a silica gel column, being eluted first with hexanes (1L) to remove the diphenyl ether, and then 3% EtOAc/hexanes to afford the desired quinoline **3B5** (2.76 g, 52%) as a pale yellow solid. The product was suitable for use in the next step.

### Step D:

To a solution of quinoline **3B5** (2.76 g crude; 8.85 mmol; 1 eq) in THF (10 mL) and methanol (10 mL) at R.T. was added 1 N NaOH (45 mL; 45 mmol; 5.1 eq). The reaction was allowed to stir at reflux (85°C) for 24 h (monitored by HPLC). The mixture was acidified with 4N HCl and extracted with methylene chloride (3X). The organic fractions were dried over MgSO₄, filtered and concentrated to afford the quinoline acid **3B6** (2.43 g, 97%) as a pale yellow solid. MS: (M + H)⁺; 284. This material was used as is for the following reaction.

### Step E:

Compound **3B6** (2.43 g, 8.56 mmol) was added to diphenyl ether (20 mL) and the heterogeneous mixture was heated to 250°C for 12 minutes before being cooled. The mixture was directly transferred to a silica gel column and eluted first with hexanes (to remove diphenyl ether), and then with 30% and 50% EtOAc/hexanes (R_{f}=0.48 in EtOAc/hexanes (1:1)). Evaporation of the solvent afforded the desired 2-thioethyl-8-chloro-4-hydroxyquinoline **3B7** (1.25 g, 61 %) as a pale yellow solid. MS: (M + H)⁺; 240, HPLC homogeneity = 99%.

### EXAMPLE 3C

### Preparation of 2-ethoxy-8-thiomethyl-4-hydroxyquinoline (3C4)

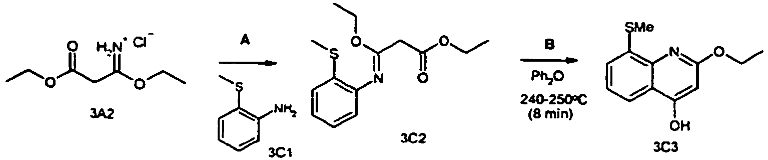

### Step A:

The imidate salt **3A2** (1.4 g, 7.2 mmol, 1 eq.) was combined with 2-(methylthio)aniline **3C1** (0.96 g, 7.50 mmol, 1 eq.) in ethanol (15 mL) under an N₂ atmosphere. The reaction mixture was stirred at R.T. (1 h) and monitored by HPLC. The reaction mixture was concentrated and then ether was added and the mixture filtered. The solids were washed with ether and the combined ether washes concentrated *in vacuo.* The resulting adduct **3C2** was obtained as a yellow oil (1.66 g, 82%) and used as is in the next step. MS electrospray: (M + H)⁺; 282 and (M - H)⁻; 280.

### Step B:

The condensation product **3C2** (1.66 g, 5.90 mmol) was dissolved in diphenyl ether (10 mL) and placed in a sand bath (300°C). The internal temperature was monitored and maintained between 240-250°C for 10 minutes. The mixture was cooled and then directly loaded on a silica gel column and eluted first with hexanes, then with 30% EtOAc/Hexanes and finally 50% EtOAc/hexanes. The product was concentrated and dried in *vacuo* to give the corresponding 4-hydroxyquinoline derivative **3C3** as a yellow solid (0.735 g, 53%). MS electrospray: (M + H)⁺; 236 and (M - H)⁻; 234.

### EXAMPLE 3D

### Preparation of 2-ethoxy-8-methoxy-4-hydroxyquinoline (3D3)

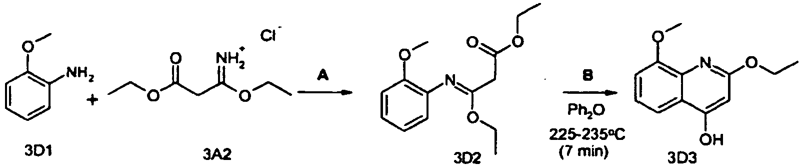

Beginning with ortho-anisidine **3D1** and following the same protocol as outlined in Example 3C, the desired 8-methoxyquinoline derivative **3D3** was obtained in 38% overall yield as a pale yellow solid. MS: 220 (M +H)⁺.

### EXAMPLE 3E

### Preparation of 2-ethoxy-8-bromo-7-methoxy-4-hydroxyquinoline (3E2)

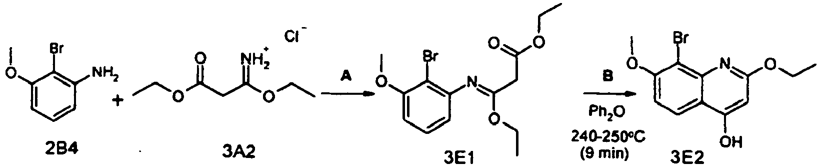

### Step A:

To 2-bromo-3-aminoanisole **2B4** (750 mg, 3.7 mmol, 1 eq) was added imidate **3A2** (0.73 g, 3.7 mmol, 1 eq) in ethanol (7 mL) under a N₂ atmosphere. The mixture was stirred at R.T. for 24 h at which point the reaction was concentrated and purified directly on a silica gel column (eluent EtOAc/Hexanes (1:9)) to afford adduct **3E1** (1.12 g, 88%) as a pale yellow oil. MS: **344** (M + H)⁺ and 346 (MH + 2)⁺.

### Step B:

Adduct **3E1** (1.12 g, 3.25 mmol) was dissolved in diphenyl ether (10 mL) and placed in a pre-heated sand bath (300°C). The internal temperature was monitored and allowed to stay between 240°C-250°C for 8 minutes. The mixture was directly loaded on a silica gel column and eluted with hexanes to remove diphenyl ether, followed by a gradient of 30% to 50% EtOAc/hexanes: (R_{f} =0.25 in 1:1 EtOAc/hexanes). Concentration and drying in *vacuo* afforded the desired quinoline **3E2** (734 mg, 76%) as a white solid. MS: 298 (M + H)⁺ and 300 (MH + 2)⁺.

### EXAMPLE 4A

### Preparation of P1' fragments Cyclopropylsulfonamide (4A4) and 1-methylcyclopropylsulfanamide (4A7)

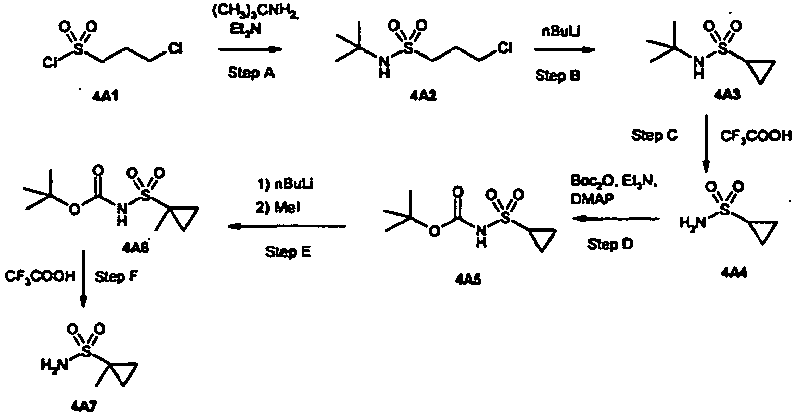

Cyclopropanesulfonamide can be prepared by amination of cyclopropanesulfonyl chloride, according to the literature reference of J. King et al., J. Org. Chem., 1993, 58, 1128-1135, or as set out below.

### Step A:

A dry 3 L 3-neck flask equipped with a magnetic stir bar, addition funnel and argon inlet was flushed with argon, then charged with 3-chloropropanesulfonyl chloride **4A1** (100.48 g, 0.57 mol, 1.0 eq). Anhydrous dichloromethane (900 mL) was transferred into the flask via cannula, the mixture was cooled in an ice/water bath and tert-butylamine (72 mL, 0.68 mol, 1.2 eq) was added. The mixture was stirred 15 minutes then a solution of triethylamine (158 mL, 1.13 mol, 2.0 eq) in anhydrous dichloromethane (100 mL) was added dropwise over 45 minutes and stirring was continued for 1 h. The mixture was diluted with dichloromethane (500 mL) and washed with 1N HCl (3 x 400 mL) and brine. The organic layer was dried over sodium sulfate, filtered and evaporated to dryness to give compound **4A2** as an orange-beige solid (107.04 g, 88% yield). ¹H NMR (CDCl₃, 400 MHz): δ **4.46** (s, 1H), 3.71 (tr, 2H), 3.25 (tr, 2H), 2.31 (m, 2H), 1.41 (s, 9H).

### Step B:

A dry 5 L 3-neck flask equipped with a magnetic stir bar, argon inlet and 2 addition funnels was flushed with argon and anhydrous THF (1.5 L) was transferred into the flask via cannula and cooled to -78°C. Compound **4A2** (96.73 g, 0.453 mol, 1.0 eq) was dissolved in anhydrous THF (390 mL) and the solution was transferred into one of the addition funnels. n-Butyllithium solution (2.5 M in hexanes, 390 mL, 0.975 mol, 2.15 eq) was transferred to the other addition funnel and the solutions in the addition funnels were added to the flask simultaneously over 4 hours. When addition was complete, the mixture was allowed to warm to room temperature. Once the internal temperature reached ∼0°C, the reaction was quenched by dropwise addition of saturated NH₄Cl solution (200 mL). The THF was removed under vacuum and the residue was diluted with CH₂Cl₂ (2 L) and water (1 L). The layers were separated and the organic layer was washed with water (2 x 1 L) and brine (800 mL), dried over sodium sulfate, filtered and evaporated to dryness. Compound **4A3** was obtained as an orange-beige solid (77.32 g, 96% yield). ¹H NMR (CDCl₃, 400 MHz): δ 4.25 (s, 1H), 2.48 (m, 1H), 1.42 (s, 9H), 1.19 (m), 1.01 (m).

### Step C:

A 2L flask equipped with a magnetic stir bar and condenser was charged with Compound **4A3** (82.53 g, 0.466 mol, 1.0 eq), dichloromethane (400 mL) and trifluoroacetic acid (460 mL, 5.97 mol, 13 eq). The mixture was heated to reflux for 2 h, allowed to cool, and evaporated and co-evaporated several times with CH₂Cl₂ to remove most of the TFA. The crude product was dissolved in 95:5 CH₂Cl₂:MeOH and NH₄OH and was purified by silica gel column chromatography (94:5:1 CH₂Cl₂:MeOH:NH₄OH). Compound **4A4** was obtained as a beige solid (46.38 g, 78% yield). ¹H NMR (DMSO-d₆, 400 MHz): δ 6.79 (s, 2H), 2.54 (1H, under DMSO peak), 0.92 (4H).

### Step D:

To the solid cyclopropanesulfonamide **4A4** (1.51 g ; 12.46 mmol) was added in sequence: di-t-butyl-dicarbonate (3.26 g ; 14.95 mmol) dissolved in anhydrous dichloromethane (15 mL), triethylamine (2.6 mL; 18.65 mmol) and dimethylaminopyridine (76 mg; 0.622 mmol). The resulting solution was stirred at room temperature overnight and subsequently evaporated to near dryness. The residue was diluted with EtOAc, washed with 1 N aq. HCl (3x) and brine (1x), dried (MgSO₄), filtered and evaporated to dryness to provide the Boccyclopropylsulfonamide product **4A5** as a white solid (2.6 g ; 94%).

### Step E:

To a cooled solution (-78°C) of the Boc-cyclopropanesulfonamide **4A5** (500 mg; 2.26 mmol) in anhydrous THF (15 mL) was added dropwise n-BuLi (2.1 mL; 5.20 mmol) and the mixture was allowed to stir 1 h at -78°C. Two portions of methyl iodide (each 280 µL; 4.52 mmol) were added with a one hour interval and the reaction mixture was allowed to warm slowly to RT and stir at RT overnight. The reaction mixture was adjusted to pH 3 with 1N aq. HCl and the product was extracted with EtOAc (3x). The combined EtOAc extracts were washed with brine (1x), dried (MgSO₄), filtered and evaporated to dryness to provide the crude alkylated product **4A6** as a light yellow oil . The crude material was purified by flash chromatography over silica gel with hexane: EtOAc (9: 1) as eluent to provide pure product as a yellow oil (151.8 mg; 29%).

### Step F:

To a solution of the Boc-1-methylcyclopropanesulfonamide **4A6** (151.8 mg: 0.65 mmol) in dichloromethane (6 mL) was added trifluoroacetic acid (6 mL) and the mixture allowed to stir at RT for 3.5 h. Evaporation to dryness under high vacuum provided the deprotected material **4A7** as an off- white wax like solid (79.1 mg, 91%). ¹H NMR (CDCl₃, 400 MHz): δ 4.56 (s, 2H), 1.58 (s, 3H), 1.43-1.38 (m, 2H), 0.85-0.80 (2H).

### EXAMPLE 4B

### Synthesis of P1-P1' fragment (4B3)

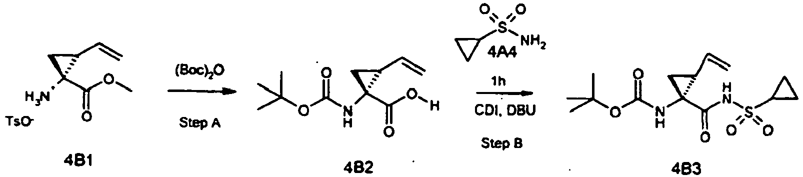

### Step A:

To a solution of compound **4B1** (12 g, 38.29 mmol) in a mixture of THF (50 mL) and 1 N aq. NaOH (85 mL, 85.00 mmol) was added Boc anhydride (10 g, 45.95 mmol). The reaction mixture was stirred at RT for 4 days. The pH was periodically adjusted to 9 by adding more NaOH. The THF was then removed *in vacuo* and the aqueous layer was washed with ether (3 X 150 mL) and then cooled to 0°C for the slow addition of 1 N aq. HCl until pH 3-4 was obtained. The aqueous layer was then extracted with EtOAc (3 X 150 mL) and the combined organic extracts were successively washed with water (3 X 100 mL) and brine. After drying over MgSO₄, filtration and concentration, 5.16 g of the desired Boc-protected intermediate **4B2** was isolated.

### Step B:

To a solution of acid **4B2** (567 mg, 2.49 mmol), in THF (20 mL), was added CDI (515 mg, 3.17 mmol). The resulting solution was stirred for 30 min, refluxed for 30 min and allowed to cool down to RT. Cyclopropylsulfonamide **4A4** (455 mg, 3.76 mmol) was added followed by the addition of DBU (0.75 mL, 5.02 mmol) and the reaction was stirred 12 h. The THF was removed in vacuo and the residue was diluted with EtOAc, washed with 1 M HCl (2 X 100 mL) and brine, dried (MgSO4) and purified by flash chromatography (elution conditions: 70:30 hexane/EtOAc) to afford 682 mg (82%) of compound **4B3** as a white solid.

### EXAMPLE 5A

### Synthesis of Compound 1003 of Table 1

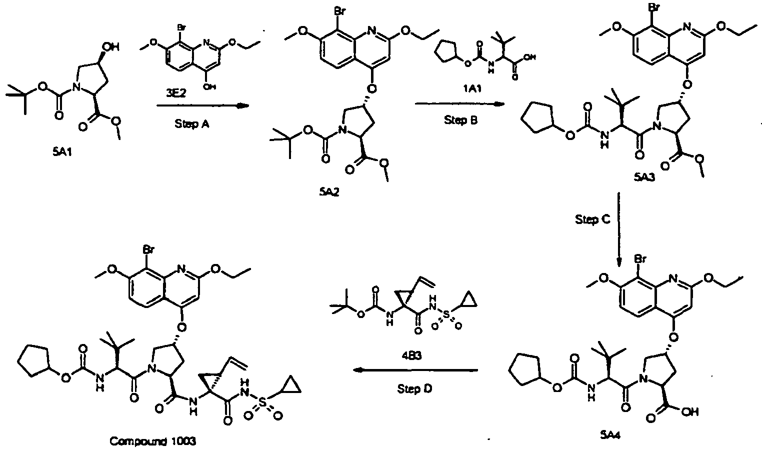

### Step A:

A solution of commercially available protected 4-hydroxyproline fragment **5A1** (75 mg, 0.306 mmol), quinoline **3E2** (137 mg, 0.460 mmol, 1.5 equiv.) and PPh₃ (160 mg, 0.610 mmol, 2.0 equiv.) in 5 mL of THF, was cooled to 0°C for the slow addition of DIAD (0.12 mL, 0.609 mmol, 2.0 equiv.). The reaction mixture was allowed to warm up to room temperature overnight. The solvent was removed *in vacuo* and the residue purified by flash chromatography (elution conditions: 70:30 Hexanes/EtOAc) to provide 90 mg (0.171 mmol, 56%) of the desired compound **5A2**.

### Step B:

A mixture of compound **5A2** (90 mg, 0.171 mmol) and 4 mL of HCl 4M/dioxane was stirred at room temperature for one hour. The solvent was removed in vacuo and the residue dried under vacuum, then dissolved in 2 mL of CH₃CN and to this solution was added 0.15 mL of DIEA (0.861 mmol, 5.0 equiv.), followed by a mixture of acid **1A1** (51 mg, 0.210 mmol, 1.22 equiv.) and HATU (81 mg, 0.213 mmol, 1.24 equiv.) in 3 mL of CH₃CN. The resulting mixture was stirred at room temperature overnight. The solvent was removed in vacuo and the residue was diluted with EtOAc and successively washed twice with saturated NaHCO₃ then with brine. After drying over MgSO₄, filtration and concentration, 133 mg (>100%) of the crude compound **5A3** was obtained.

### Step C:

To the ester **5A3** (111 mg, 0.171 mmol), in 3.5 mL of a mixture THF:H₂O (2.5:1), was added LiOH.H₂O (18 mg, 1.60 mmol, 1.5 equiv.) followed by 1 mL of MeOH. The resulting solution was stirred at room temperature for 2 hours. The solvents were removed in vacuo and the residue was diluted with H₂O, acidified with 1M HCl to pH 5.5 and then extracted twice with EtOAc. After drying over MgSO₄, filtration and concentration, 103 mg (0.162 mmol, 94%) of acid **5A4** was isolated.

### Step D:

Compound **4B3** (20 mg, 0.061 mmol, 1.07 equiv.), in 2 mL of HCl 4M/dioxane, was stirred at room temperature for 75 minutes. The solvent was removed in vacuo and the residue was dried under vacuum, then dissolved in DMF (1.5 mL) and to this solution was added DIEA (0.050 mL, 0.287 mmol, 5.08 equiv.). This mixture was added to a mixture of the acid **5A4** (36 mg, 0.057 mmol) and HATU (25 mg, 0.066 mmol, 1.16 equiv.) in 0.5 mL of DMF and the resulting solution was stirred at room temperature overnight. The reaction mixture was diluted with AcOH and purified by preparatory HPLC. The pure fractions were combined, concentrated and lyophilized to provide 25 mg of the desired compound **1003** (52%).
¹H NMR (400 MHz,DMSO-d₆): ca, 95: 5 mixture of rotamers, major isomer description; δ 10.46 (s, 1H), 8.80 (s, 1H), 7.98 (d, J = 9.0 Hz, 1H), 7.18 (d, J = 9.2 Hz, 1H), 6.97 (d, J = 8.2 Hz, 1H), 6.45 (s, 1H), 5.65-5.54 (m, 1H), 5.43-5.39 (m, 1H), 5.25-5.18 (m, 1H), 5.11-5.06 (m, 1H), 4.59-4.53 (m, 1H), 4.51 (q, J = 7.0 Hz, 2H), 4.39-4.31 (m, 2H), 4.08-4.03 (m, 1H), 3.95 (s, 3H), 3.93-3.86 (m, 1H), 2.96-2.88 (m, 1H), 2.56-2.48 (m, 1H), 2.18-2.08 (m, 2H), 1.72-1.61 (m, 2H), 1.61-1.20 (m, 8H), 1.39 (t, J = 7.0 Hz, 3H), 1.11-0.98 (m, 4H), 0.95 (s, 9H). M.S.(electrospray) : 846 and 848.2 (M-H)⁻ 848 and 850.2 (M+H)⁺. Reverse Phase HPLC Homogeneity (0.06 % TFA; CH₃CN : H₂O) : 99 %

### EXAMPLE 5B

### Synthesis of Compound 1010 of Table 1:

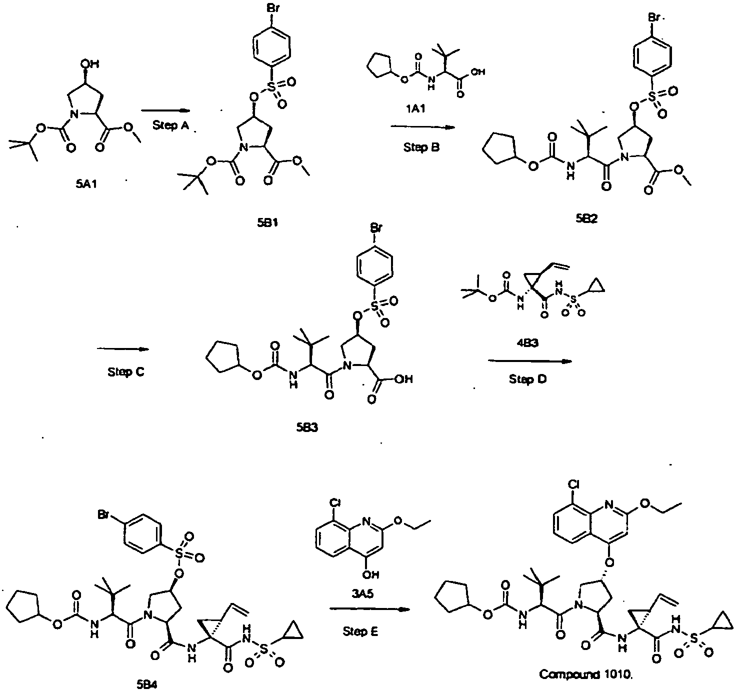

### Step A:

To a solution of commercially available protected 4-hydroxyproline fragment **5A1** (2.0 g, 8.15 mmol, 1 equiv.), brosyl chloride (4.17 g, 16.32 mmol, 2.0 equiv.) and DMAP (0.10 g, 0.81 mmol, 0.1 equiv.), dissolved in dichloromethane (75 mL), was added triethylamine (4.0 mL, 28.70 mmol, 3.5 equiv.). The resulting solution was allowed to stir at 40°C overnight and the reaction mixture was concentrated to dryness. The residue was diluted with EtOAc, washed twice with 1 M HCl, twice with saturated sodium bicarbonate and once with brine, dried over MgSO₄, filtered and concentrated under vacuum. The crude material was purified by flash column chromatography with hexanes/EtOAc; 75:25 to provide 3.5 g of the desired compound **5B1** (92% yield).

### Step B:

To compound **5B1** (3.5 g, 7.54 mmol), in 20 mL of CH₂Cl₂, was added 5 mL of TFA and the resulting solution was stirred at RT for 3 hours, then concentrated to dryness. The residue was dissolved in CH₃CN (15 mL) and DIEA (6.6 mL, 37.89 mmol, 5.0 equiv.) was added. This mixture was added to a mixture of acid **1A1** (2.12 g, 8.71 mmol, 1.15 equiv.) and HATU (3.43 g, 9.02 mmol, 1.2 equiv.) in 25 mL of CH₃CN and the resulting solution was allowed to react at RT overnight, then was concentrated to dryness. The residue was diluted with EtOAc, washed twice with saturated sodium bicarbonate and once with brine, dried over MgSO₄, filtered and concentrated under vacuum. The crude material was purified by flash column chromatography with hexanes/EtOAc; 70:30 to provide 3.46 g of the desired compound **5B2** (78% yield).

### Step C:

To a solution of compound **5B2** (102 mg, 0.173 mmol) in 3 mL of a mixture of THF:H₂O (2.5:1), was added 0.26 mL of 1 M NaOH (0.26 mmol, 1.5 equiv.), followed by MeOH (0.5 mL). The mixture was stirred at RT overnight and the solvents were removed under vacuum. The residue was diluted with H₂O and acidified with 1M HCl. The aqueous layer was extracted three times with EtOAc, and the organic extract was dried over MgSO₄, filtered and concentrated under vacuum to give 90 mg (90% yield) of the crude material **5B3.**

### Step D:

Compound **4B3** (307 mg, 0.929 mmol, 1.05 equiv.) was dissolved in 5 mL of 4M HCl/dioxane and the resulting solution was stirred at RT for 1.5 h and concentrated to dryness. The residue was dissolved in DMF (4 mL) and DIEA (0.75 mL, 4.30 mmol, 4.9 equiv.) was added. This mixture was added to a mixture of acid **5B3** (506 mg, 0.879 mmol) and HATU (351 mg, 0.923 mmol, 1.05 equiv.) in 3 mL of DMF and the resulting solution was allowed to react at RT for 6 hours. The reaction mixture was diluted with EtOAc, washed with H₂O and brine, dried over MgSO₄, filtered and concentrated under vacuum. The crude material was purified by flash column chromatography with Hexanes/EtOAc; 35:65 to provide 385 mg of the desired compound **5B4** (55% yield).

### Step E:

A mixture of the brosylate tripeptide **5B4** (35 mg, 0.044 mmol), quinoline **3A5** (14 mg. 0.063 mmol, 1.41 equiv.) and cesium carbonate (31 mg, 0.095 mmol, 2.14 equiv.), in 1.5 mL of DMF, was heated at 70-72°C for 4 hours. The reaction mixture was diluted with AcOH and purified by preparatory HPLC. The pure fractions were combined, concentrated and lyophilized to provide 24 mg of the desired compound **1010** (69%). ¹H NMR (400 MHz,DMSO-d₆): ca, 95:5 mixture of rotamers, maior isomer description; δ 10.43 (s, 1H), 8.79 (s, 1H), 7.98 (d, J = 8.0 Hz, 1H), 7.81 (d, J = 7.2 Hz, 1H), 7.27 (t, J = 7.9 Hz, 1H), 6.96 (d, J = 8.3 Hz, 1H), 6.62 (s, 1H), 5.65-5.54 (m, 1H), 5.46-5.41 (m, 1H), 5.25-5.17 (m, 1H), 5.12-5.06 (m, 1H), 4.63-4.56 (m, 1H), 4.51 (q, J = 7.1 Hz, 2H), 4.42-4.31 (m, 2H), 4.10-3.87 (m, underwater, 2H), 2.96-2.87 (m, 1H), 2.57-2.48 (m, 1H), 2.18-2.08 (m, 2H), 1.72-1.62 (m, 2H), 1.62-1.23 (m, 8H), 1.40 (t, J = 7.1 Hz, 3H), 1.11-0.98 (m, 4H), 0.95 (s, 9H). M.S.(electrospray) : 772.2 (M-H)- 774.3 (M+H)+ . Reverse Phase HPLC Homogeneity (0.06 % TFA; CH₃CN : H₂O): 99 %

### EXAMPLE 6

### NS3-NS4A protease assay

The enzymatic assay used to evaluate the present compounds is described in WO 00/09543 and WO 00/59929.

### EXAMPLE 7

### Cell Based HCV RNA Replication Assays

The cell-based HCV RNA replication assays used to evaluate the present compounds are described in detail in WO 2005/028501 and in WO 2004/103996.

Representative compounds of this invention are found to show unexpectedly good activity in the preceding enzymatic and cell based assays.

### EXAMPLE 8

### Specificity assays

The specificity assays used to evaluate the selectivity of this compound are described in WO 00/09543.

Representative compounds of this invention are found to be selective in that they do not show significant inhibition (no measurable activity at concentrations up to 30 µM) in the Human Leukocyte Elastase and Cathepsin B assays.

### TABLE OF COMPOUNDS

The following table list compounds representative of the invention. Most of the compounds listed in Table 1 below were tested in the NS3-NS4A protease enzymatic assay described in Example 6 and/or in the cell-based HCV RNA replication assay described in Example 7 and were found to have unexpectedly good activity. Retention times (t_{R}) for each compound were measured using the standard analytical HPLC conditions described in the Examples. As is well known to one skilled in the art, retention time values are sensitive to the specific measurement conditions. Therefore, even if identical conditions of solvent, flow rate, linear gradient, and the like are used, the retention time values may vary when measured, for example, on different HPLC instruments. Even when measured on the same instrument, the values may vary when measured, for example, using different individual HPLC columns, or, when measured on the same instrument and the same individual column, the values may vary, for example, between individual measurements taken on different occasions. **TABLE 1**

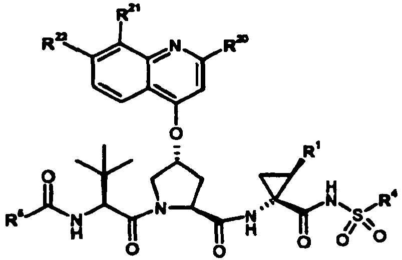

| **Cpd** | **R⁵** | **R²²** | **R²¹** | **R²⁰** | **R¹** | **R⁴** | **t_{R} (min)** | **MS (M+H)⁺** |
|---|---|---|---|---|---|---|---|---|
| **1001** | 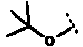 | OMe | H | OEt | 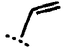 | 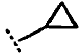 | 5.9 | 758.4 |
| **1002** | 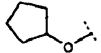 | H | Me | OEt | 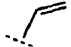 | 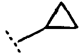 | 7.2 | 754.4 |
| **1003** | 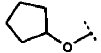 | OMe | Br | OEt | 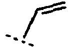 | 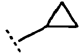 | 7.7 | 848.2 850.2 |
| **1004** | 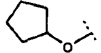 | OMe | Br | OEt | 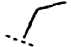 | 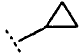 | 7.8 | 850.0 852.3 |
| **1005** | 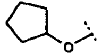 | H | Me | OEt | 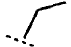 | 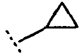 | 7.2 | 756.3 |
| **1006** | 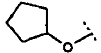 | OMe | Br | OEt | 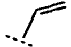 | 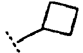 | 8.0 | 860.1 862.1 |
| **1007** | 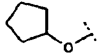 | H | SMe | OEt | 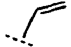 | 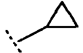 | 7.7 | 786.3 |
| **1008** | 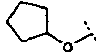 | H | SMe | OEt | 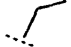 | 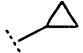 | 7.8 | 788.2 |
| **1009** | 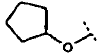 | H | Cl | OMe | 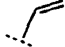 | 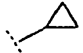 | 7.6 | 760.2 |
| **1010** | 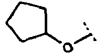 | H | Cl | OEt | 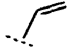 | 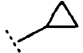 | 7.8 | 774.3 |
| **1011** | 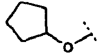 | H | Cl | SEt | 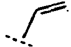 | 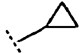 | 8.0 | 790.3 |
| **1012** | 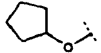 | OMe | Me | OMe | 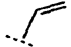 | 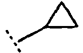 | 6.2 | 770.3 |
| **1013** | 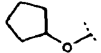 | OMe | Me | OEt | 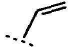 | 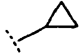 | 6.4 | 784.4 |
| **1014** | 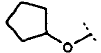 | H | Cl | OMe | 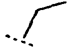 | 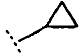 | 7.2 | 762.3 |
| **1015** | 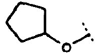 | H | Cl | OEt | 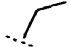 | 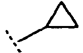 | 7.5 | 776.3 |
| **1016** | 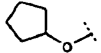 | OMe | Me | OMe | 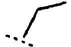 | 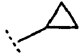 | 5.9 | 772.4 |
| **1017** | 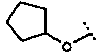 | OMe | Me | OEt | 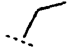 | 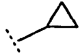 | 6.1 | 786.4 |
| **1018** | 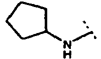 | OMe | Br | OEt | 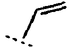 | 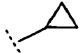 | 7.4 | 847 849 |
| **1019** | 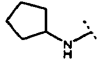 | OMe | Me | OEt | 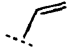 | 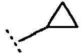 | 6.1 | 783 |
| **1020** | 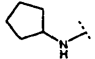 | H | Cl | OEt | 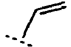 | 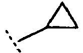 | 7.7 | 773 771 |
| **1021** | 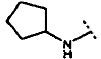 | H | Me | OEt | 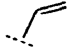 | 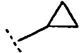 | 6.5 | 753 |
| **1022** | 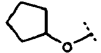 | H | OMe | OEt | 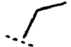 | 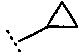 | 5.2 | 772.3 |
| **1023** | 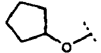 | H | Br | OEt | 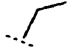 | 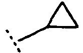 | 7.5 | 820.2 822.2 |
| **1024** | 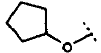 | OMe | Br | OEt | 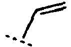 | 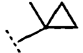 | | |
| **1025** | 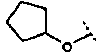 | H | Cl | OEt | 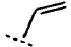 | 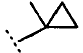 | | |
| **1026** | 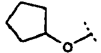 | OMe | Me | OEt | 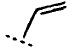 | 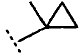 | | |
| **1027** | 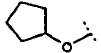 | H | SMe | OEt | 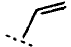 | 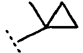 | | |

## Claims

1. A compound of formula (I): 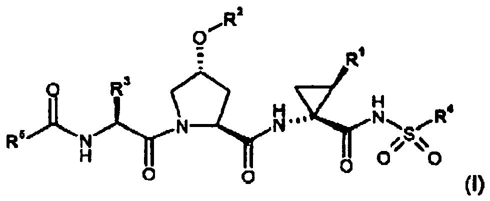 wherein
**R¹** is selected from (C₂₋₄)alkenyl and (C₂₋₄)alkyl;
**R²** is a group of formula: 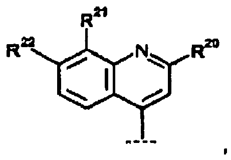 wherein
**R²⁰** is selected from -O-(C₁₋₆)alkyl and -S-(C₁₋₆)alkyl;
**R²¹** is selected from H, (C₁₋₆)alkyl, halogen, -O-(C₁₋₆)alkyl and -S-(C₁₋₆)alkyl; and
**R²²** is H or -O-(C₁₋₄)alkyl;
**R³** is (C₁₋₆)alkyl;
**R⁴** is (C₃₋₈)cycloalkyl, optionally substituted with (C₁₋₄)alkyl; and
**R⁵** is **R⁵⁰**-O- or **R⁵⁰**-NH-; wherein **R⁵⁰** is (C₁₋₆)alkyl or (C₃₋₇)cycloalkyl; wherein the (C₃₋₇)cycloalkyl is optionally substituted with (C₁₋₆)alkyl;
or a salt thereof.

2. A compound according to claim 1 wherein **R¹** is selected from ethenyl and ethyl.

3. A compound according to claim 1 wherein **R²⁰** is methoxy, ethoxy, propoxy, 1-methylethoxy, methylthio, ethylthio, propylthio or 1-methylethylthio; **R²¹** is H, fluoro, chloro, bromo, methyl, ethyl, methoxy, ethoxy, methylthio or ethylthio; and **R²²** is H, methoxy or ethoxy.

4. A compound according to one or more of claims 1 to 3 wherein **R³** is 1,1-dimethylethyl.

5. A compound according to one or more of claims 1 to 4 wherein **R⁴** is cyclopropyl or cyclobutyl, each of which being optionally substituted with methyl, ethyl, propyl or 1-methylethyl.

6. A compound according to one or more of claims 1 to 5 wherein **R⁶** is **R⁵⁰**-O-, wherein **R⁵⁰** is defined as in claim 1.

7. A compound according to claim 6 wherein **R⁵⁰** is selected from 1,1-dimethylethyl and cyclopentyl.

8. A compound according to one or more of claims 1 to 7 wherein **R⁵** is **R⁵⁰**-NH-, wherein **R⁵⁰** is defined as in claim 1.

9. A compound according to claim 8 wherein **R⁵⁰** is selected from 1,1-dimethylethyl and cyclopentyl.

10. A compound according to one or more of claims 1 to 9, or a pharmaceutically acceptable salt thereof; as a medicament.

11. A pharmaceutical composition comprising a therapeutically effective amount of a compound according to one or more of claims 1 to 9, or a pharmaceutically acceptable salt thereof; and one or more pharmaceutically acceptable carriers.

12. The pharmaceutical composition according to claim 11 additionally comprising at least one other antiviral agent.

13. Use of a compound according to one or more of claims 1 to 9, or a pharmaceutically acceptable salt thereof, for the manufacture of a pharmaceutical composition for the treatment of a hepatitis C viral infection in a mammal having or at risk of having the infection.

14. Use of a compound according to one or more of claims 1 to 9, or a pharmaceutically acceptable salt thereof and at least one other antiviral agent for the manufacture of a pharmaceutical composition for the treatment of a hepatitis C viral infection in a mammal having or at risk of having the infection.

15. An article of manufacture comprising a composition effective to treat a hepatitis C viral infection or to inhibit the NS3 protease of HCV; and packaging material comprising a label which indicates that the composition can be used to treat infection by the hepatitis C virus; wherein the composition comprises a compound according to one or more of claims 1 to 9 or a pharmaceutically acceptable salt thereof.

## Patentansprüche

1. Verbindung der Formel (I) 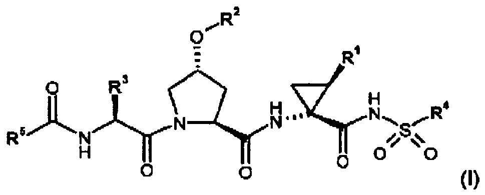 worin
**R¹** ausgewählt ist aus (C₂₋₄)-Alkenyl und (C₂₋₄)-Alkyl;
**R²** ist eine Gruppe der Formel: 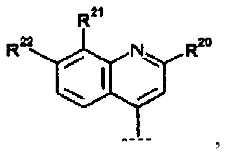 worin
**R²⁰** ausgewählt ist aus -O-(C₁₋₆)-Alkyl und -S-(C₁₋₆)-Alkyl;
**R²¹** ausgewählt ist aus H, (C₁₋₆)-Alkyl, Halogen, -O-(C₁₋₆)-Alkyl und -S-(C₁₋₆)-Alkyl; und
**R²²** ist H oder -O-(C₁₋₄)-Alkyl,
**R³** ist (C₁₋₆)-Alkyl;
**R⁴** ist (C₃₋₈)-Cycloalkyl, gegebenenfalls substituiert mit (C₁₋₆)-Alkyl, und
**R⁵** ist **R⁵⁰**-O- oder **R⁵⁰**-NH-, worin **R⁵⁰** (C₁₋₆)-Alkyl oder (C₃₋₇)-Cycloalkyl darstellt;
wobei das (C₃₋₇)-Cycloalkyl gegebenenfalls substituiert ist mit (C₁₋₆)-Alkyl; oder ein Salz davon.

2. Verbindung nach Anspruch 1, worin **R¹** ausgewählt ist aus Ethenyl und Ethyl.

3. Verbindung nach Anspruch 1, wobei **R²⁰** Methoxy, Ethoxy, Propoxy, 1-Methylethoxy, Methylthio, Ethylthio, Propylthio oder 1-Methylethylthio darstellt; **R²¹** ist H, Fluor, Chlor, Brom, Methyl, Ethyl, Methoxy, Ethoxy, Methylthio oder Ethylthio; und **R²²** ist H, Methoxy oder Ethoxy.

4. Verbindung nach einem oder mehreren der Ansprüche 1 bis 3, worin **R³** 1,1-Dimethylethyl darstellt.

5. Verbindung nach einem oder mehreren der Ansprüche 1 bis 4, worin **R⁴** Cyclopropyl oder Cyclobutyl darstellt, von denen jedes gegebenenfalls substituiert ist mit Methyl, Ethyl, Propyl oder 1-Methylethyl.

6. Verbindung nach einem oder mehreren der Ansprüche 1 bis 5, worin **R⁵ R⁵⁰**-O- darstellt, wobei **R⁵⁰** definiert ist wie in Anspruch 1.

7. Verbindung nach Anspruch 6, wobei **R⁵⁰** ausgewählt ist aus 1,1-Dimethylethyl und Cyclopentyl.

8. Verbindung nach einem oder mehreren der Ansprüche 1 bis 7, wobei R⁵ **R⁵⁰**-NH- darstellt, wobei **R⁵⁰** definiert ist wie in Anspruch 1.

9. Verbindung nach Anspruch 8, wobei **R⁵⁰** ausgewählt ist aus 1,1-Dimethylethyl und Cyclopentyl.

10. Verbindung nach einem oder mehreren der Ansprüche 1 bis 9 oder ein pharmazeutisch akzeptables Salz davon als Arzneimittel.

11. Pharmazeutische Zusammensetzung, umfassend eine therapeutisch wirksame Menge einer Verbindung nach irgendeinem oder mehreren der Ansprüche 1 bis 9 oder eines pharmazeutisch akzeptablen Salzes davon und ein oder mehreren pharmazeutisch akzeptablen Trägem.

12. Pharmazeutische Zusammensetzung nach Anspuch 11, zusätzlich umfassend mindestens ein anderes antivirales Mittel.

13. Verwendung einer Verbindung nach einem oder mehreren der Ansprüche 1 bis 9 oder eines pharmazeutisch akzeptablen Salzes davon zur Herstellung einer pharmazeutischen Zusammensetzung zur Behandlung einer viralen Hepatitis-C-Infektion in einem Säuger mit der Infektion oder dem Risiko, eine Infektion aufzuweisen.

14. Verwendung einer Verbindung nach einem oder mehreren der Ansprüche 1 bis 9 oder eines pharmazeutisch akzeptablen Salzes davon und mindestens einem anderen antiviralen Mittel zur Herstellung einer pharmazeutischen Zusammensetzung zur Behandlung einer viralen Hepatitis-C-Infektion in einem Säuger mit der Infektion oder dem Risiko, die Infektion aufzuweisen.

15. Herstellungsartikel bzw. Produkt, umfassend eine Zusammensetzung, die wirksam ist zur Behandlung einer viralen Hepatitis-C-Infektion oder zur Inhibierung der NS3-Protease von HCV; und Verpackungsmaterial, umfassend eine Kennzeichnung, die angibt, dass die Zusammensetzung zur Behandlung einer Infektion durch das Hepatitis-C-Virus verwendet werden kann; wobei die Zusammensetzung eine Verbindung nach einem oder mehreren der Ansprüche 1 bis 9 oder ein pharmazeutisch akzeptables Salz davon umfasst.

## Revendications

1. Composé de formule (I) : 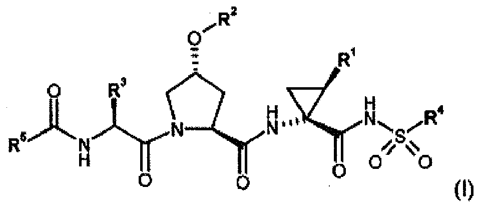 dans laquelle
R¹ est choisi parmi le groupe alcényle en C₂-C₄ et le groupe alkyle en C₂-C₄ ;
R² est un groupe de formule : 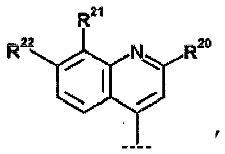 dans laquelle
R²⁰ est choisi parmi un groupe -O-alkyle en C₁-C₆ et un groupe -S-alkyle en C₁-C₆ ;
R²¹ est choisi parmi H, un groupe alkyle en C₁-C₆, l'halogène, un groupe -O-alkyle en C₁-C₆ et un groupe -S-alkyle en C₁-C₆ ; et
R²² est H ou un groupe -O-alkyle en C₁-C₄ ;
R³ est un groupe alkyle en C₁-C₆ ;
R⁴ est un groupe cycloalkyle en C₃-C₆, éventuellement substitué par un groupe alkyle en C₁-C₆ ; et
R⁵ est un groupe R⁵⁰-O- ou un groupe R⁵⁰-NH- ; dans lequel R⁵⁰ est un groupe alkyle en C₁-C₆ ou un groupe cycloalkyle en C₃-C₇ ; le groupe cycloalkyle en C₃-C₇ étant éventuellement substitué par un groupe alkyle en C₁-C₆ ; ou un sel de celui-ci.

2. Composé selon la revendication 1, dans lequel R¹ est choisi parmi le groupe éthényle et le groupe éthyle.

3. Composé selon la revendication 1, dans lequel R²⁰ est un groupe méthoxy, éthoxy, propoxy, 1-méthyléthoxy, méthylthio, éthylthio, propylthio ou 1-méthyléthylthio ; R²¹ est H, un groupe fluoro, chloro, bromo, méthyle, éthyle, méthoxy, éthoxy, méthylthio ou éthylthio ; et R²² est H, un groupe méthoxy ou éthoxy.

4. Composé selon une ou plusieurs des revendications 1 à 3, dans lequel R³ est un groupe 1,1-diméthyléthyle.

5. Composé selon une ou plusieurs des revendications 1 à 4, dans lequel R⁴ est un groupe cyclopropyle ou un groupe cyclobutyle, chacun étant éventuellement substitué par un groupe méthyle, éthyle, propyle ou 1-méthyléthyle.

6. Composé selon une ou plusieurs des revendications 1 à 5, dans lequel R⁵ est un groupe R⁵⁰-O-, où R⁵⁰ est défini comme dans la revendication 1.

7. Composé selon la revendication 6, dans lequel R⁵⁰ est choisi parmi un groupe 1,1-diméthyléthyle et un groupe cyclopentyle.

8. Composé selon une ou plusieurs des revendications 1 à 7, dans lequel R⁵ est un groupe R⁵⁰-NH-, où R⁵⁰ est défini comme dans la revendication 1.

9. Composé selon la revendication 8, dans lequel R⁵⁰ est choisi parmi un groupe 1,1-diméthyléthyle et un groupe cyclopentyle.

10. Composé selon une ou plusieurs des revendications 1 à 9, ou sel pharmaceutiquement acceptable de celui-ci, sous forme d'un médicament.

11. Composition pharmaceutique comprenant une quantité thérapeutiquement efficace d'un composé selon une ou plusieurs des revendications 1 à 9, ou d'un sel pharmaceutiquement acceptable de celui-ci ; et un ou plusieurs supports pharmaceutiquement acceptables.

12. Composition pharmaceutique selon la revendication 11, comprenant en outre au moins un autre agent antiviral.

13. Utilisation d'un composé selon une ou plusieurs des revendications 1 à 9, ou d'un sel pharmaceutiquement acceptable de celui-ci, pour la fabrication d'une composition pharmaceutique pour le traitement d'une infection par le virus de l'hépatite C chez un mammifère présentant l'infection ou risquant de la contracter.

14. Utilisation d'un composé selon une ou plusieurs des revendications 1 à 9, ou d'un sel pharmaceutiquement acceptable de celui-ci et d'au moins un autre agent antiviral pour la fabrication d'une composition pharmaceutique pour le traitement d'une infection par le virus de l'hépatite C chez un mammifère présentant l'infection ou risquant de la contracter.

15. Article de fabrication comprenant une composition efficace pour traiter une infection par le virus de l'hépatite C ou pour inhiber la protéase NS3 du VHC ; et un matériau d'emballage comprenant une étiquette qui indique que la composition peut être utilisée pour traiter une infection par le virus de l'hépatite C ; dans lequel la composition comprend un composé selon une ou plusieurs des revendications 1 à 9 ou un sel pharmaceutiquement acceptable de celui-ci.
